# FASCICULE DE BREVET EUROPEEN

(11) **EP 0 773 956 B1**
(45) Date de publication et mention de la délivrance du brevet: **04.07.2001**
(21) Numéro de dépôt: 95926996.0
(22) Date de dépôt: 31.07.1995
(51) Int. Cl.: C07K 5/083, A61K 38/06

(54) **FORME PURIFIEE DE STREPTOGRAMINES, SA PREPARATION ET LES COMPOSITIONS PHARMACEUTIQUES QUI LA CONTIENNENT**
STREPTOGRAMINE IN GEREINIGTER FORM, IHRE HERSTELLUNG UND DIESE ENTHALTENDE PHARMAZEUTISCHE ZUSAMMENSETZUNGEN
PURIFIED FORM OF STREPTOGRAMINES, PREPARATION OF SAME AND PHARMACEUTICAL COMPOSITIONS CONTAINING SAME

(30) Priorité: 02.08.1994 FR 9409564
(43) Date de publication de la demande: 21.05.1997
(73) Titulaire: Aventis Pharma S.A., 92160 Antony (FR)
(72) Inventeur: ANGER, Pascal, F-91370 Verrières-le-Buisson (FR); BARRIERE, Jean-Claude, F-91440 Bures-sur-Yvette (FR); BONNAVAUD, Bertrand, F-78220 Viroflay (FR); LEFEVRE, Patrick, F-94300 Vincennes (FR); PARIS, Jean-Marc, F-77360 Vaires-sur-Marne (FR); THIBAUT, Denis, F-75013 Paris (FR)
(86) Numéro de dépôt international: FR9501026
(87) Numéro de publication internationale: WO9604298

(56) Documents cités:
- EP-A- 0 506 561
- EP-A- 0 614 910
- CHEMICAL ABSTRACTS, vol. 110, no. 7, 13 Février 1989 Columbus, Ohio, US; abstract no. 56082, page 551; colonne r; & BULLETIN DES SOCIETE CHIMIQUE BELGES, vol. 97, no. 3, pages 185-192, N.K. SHARMA ET AL. 'Isolation of factor A (virginamycin M1) and factor B (mixture of VS1 and VS4) from a commercial feed additive formulation'
- BULLETIN DE LA SOCIETE CHIMIQUE DE FRANCE, vol. 2, pages 585-591, J. PREUD'HOMME ET AL. 'Pristinamycine isolement, caracterisation et identification des constituants' cité dans la demande

## Description

La présente invention concerne une forme purifiée de streptogramines comprenant au moins une composante du groupe B des streptogramines définie ci-après par la formule générale (I) cocristallisée avec au moins une composante "minoritaire" du groupe A définie ci-après par la formule générale (II).

Parmi les streptogramines connues, la pristinamycine (RP 7293), antibactérien d'origine naturelle produit par *Streptomyces pristinaes-piralis* a été isolée pour la première fois en 1955. La pristinamycine commercialisée sous le nom de Pyostacine® est constituée principalement de pristinamycine IA et de pristinamycine IIA.

Un autre antibactérien de la classe des streptogramines : la virginiamycine, a été préparé à partir de *Streptomyces virginiae*, ATCC 13161 [Antibiotics and Chemotherapy, 5, 632 (1955)]. La virginiamycine (Staphylomycine®) est constituée principalement de facteur S et de facteur M₁.

Dans le brevet US 3 325 359 ont été décrites des compositions pharmaceutiques comprenant des substances antibiotiques constituant l'antibiotique 899 : facteur S et facteur M1.

Les antibactériens d'origine naturelle, de la classe des streptogramines sont constitués du mélange de 2 groupes de composants : composantes du groupe B et composantes du groupe A, chaque groupe ayant une activité antibactérienne propre. Il a été démontré que l'association formée par les 2 groupes des composantes provoque une synergie d'action qui résulte dans une activité bactériostatique et bactéricide accrue et dans l'élargissement du spectre d'activité.

Dans Streptogramine als Modelsysteme für den Kationentransport durch Membranen, Dissertation zur Erlangung des Doktorgrades der Mathematisch-Naturwissenschaftlichen Facultät der Georg-August Universität zu Göttingen, Göttingen 1979, dans Antibiotics III, 521 (1975) et dans Antibiotics of the virginiamycin family, Inhibitors which contain synergistic components, C. Cocito, Microbiological Reviews, 145-98 (1979) ont été décrites des composantes des groupes A et B des streptogramines. J. Preud'Homme, P. Tarridec, et A. Belloc, Bull. Soc. Chim. Fr., 2, 585 (1968) ont également décrit la pristinamycine naturelle ainsi que les différentes composantes qui la constituent.

En ce qui concerne la préparation industrielle de produits de cette classe, jusqu'à présent les techniques à disposition n'avaient pas permis d'obtenir, à une échelle préparative, une forme suffisamment purifiée et la production de lots de qualité suffisamment constante et reproductible pour répondre aux exigences des législations de certains pays en matière d'enregistrement.

Dans le domaine des antibactériens, il est bien connu des praticiens que des allergies ou des résistances peuvent se développer après administration de certaines classes d'antibiotiques [The New England Journal of Medicine, 324 (9), 601 (1991)]. En milieu hospitalier on connaît notamment de nombreuses souches résistantes de Staphylococcus aureus. C'est pourquoi il est extrêmement utile pour le médecin d'avoir à sa disposition un large éventail de classes chimiquement différentes de manière à pouvoir adapter le traitement au cas particulier du malade à traiter. La conséquence de l'absence de commercialisation d'une classe déterminée peut être très grave, voire dramatique puisqu'elle peut résulter dans la privation de traitement chez des malades ne tolérant pas les autres classes d'antibiotiques.

Ainsi, les essais de purification mis en oeuvre avaient toujours eu pour but d'éliminer les composantes minoritaires des streptogramines, celles-ci étant considérées comme non indispensables et plutôt comme des impuretés.

Parmi les composantes du groupe A des streptogramines naturelles, la pristinamycine IIB (PIIB) est une composante minoritaire dont la proportion en poids est inférieure à 10 % dans la pristinamycine naturelle, et le plus souvent de l'ordre de 8 % ou même de l'ordre de 6 % dans la virginiamycine.

Il a été trouvé maintenant, et c'est ce qui fait l'objet de la présente invention, que l'association constituée d'une ou plusieurs composantes du groupe B, de formule générale : 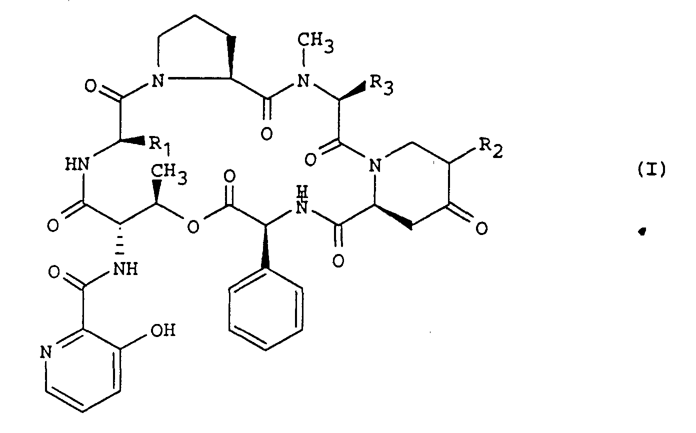 dans laquelle
- le symbole R₁ représente un radical méthyle ou éthyle,
- le symbole R₂ représente un atome d'hydrogène ou un radical hydroxy, et
- le symbole R₃ représente un radical benzyle substitué de formule générale : 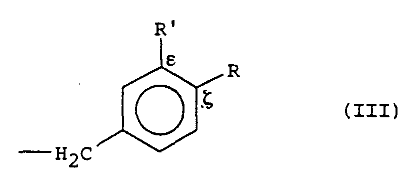
dans laquelle
1) à condition que R₂ soit simultanément hydrogène, R représente NR₄R₅ pour lequel les symboles R₄ et R₅ sont l'un un atome d'hydrogène ou un radical méthyle et l'autre un radical méthyle et R' est un atome de chlore ou de brome, ou représente un radical alcényle contenant 3 à 5 atomes de carbone si R₄ et R₅ sont des radicaux méthyle, ou bien
2) R est un atome d'hydrogène et R' représente un atome d'halogène, un radical alcoylamino ou dialcoylamino, le reste d'un éther-oxyde, un radical alcoylthio, alcoyle contenant 1 à 3 atomes de carbone en chaîne droite ou ramifiée ou trihalogénométhyle, ou bien
   R est un atome d'halogène, un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical alcényl alcoyl amino dont la partie alcényle contient 3 ou 4 atomes de carbone et la partie alcoyle est définie comme ci-dessus, un radical dialcénylamino dont les parties alcényle sont définies comme ci-dessus, un radical alcoyl cycloalcoylméthyl amino dont la partie alcoyle est définie comme ci-dessus et la partie cycloalcoyle contient 3 ou 4 atomes de carbone, le reste d'un éther-oxyde, un radical alcoylthio, un radical alcoylthiométhyle, alcoyle contennant 1 à 6 atomes de carbone en chaîne droite ou ramifiée, aryle ou trihalogénométhyle, et R' est un atome d'hydrogène, ou bien
   R est un atome d'halogène, un radical amino, alcoylamino ou dialcoylamino, le reste d'un éther-oxyde, un radical alcoylthio, alcoyle contenant 1 à 6 atomes de carbone en chaîne droite ou ramifiée ou trihalogénométhyle et R' représente un atome d'halogène, un radical alcoylamino ou dialcoylamino, le reste d'un éther-oxyde ou un radical alcoylthio ou alcoyle contenant 1 à 3 atomes de carbone en chaîne droite ou ramifiée,
   et d'une ou plusieurs composantes "minoritaires" du groupe A des streptogramines, de formule générale : 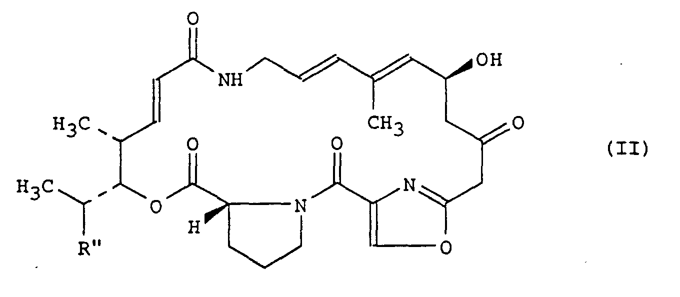 dans laquelle R" est un atome d'hydrogène ou un radical méthyle ou éthyle, est particulièrement intéressante du fait de son activité biologique in vivo.

Dans la formule générale (I), lorsque R ou R' (dans R₃) représente un atome d'halogène, il s'agit de préférence d'un atome de fluor, sauf si R' est l'atome d'hydrogène, dans ce cas R est indifféremment chlore, brome, fluor ou iode ; lorsque R' représente un radical alcoylamino ou dialcoylamino, les radicaux alcoyle sont de préférence choisis parmi méthyle ou éthyle ; lorsque R ou R' représente le reste d'un éther-oxyde, il peut être représenté par un reste OR° pour lequel R° est choisi de préférence parmi les groupements méthyle, éthyle éventuellement substitué par un atome de chlore, allyle ou trifluorométhyle ; lorsque R ou R' représente un radical alcoylthio il s'agit de préférence du radical méthylthio ; lorsque R représente un radical alcoyle contenant 1 à 6 atomes de carbone, il représente de préférence méthyle, isopropyle ou tert-butyle ; lorsque R représente un radical aryle il s'agit de préférence d'un radical phényle ; lorsque R ou R' représente un radical trihalogénométhyle il s'agit de préférence du radical trifluorométhyle. Par ailleurs il est entendu que, sauf mention spéciale, les radicaux alcoyle sont droits ou ramifiés et contiennent 1 à 4 atomes de carbone.

Le produit de formule générale (II) pour lequel R" est un radical éthyle ci-après appelé pristinamycine IIF (PIIF) et le produit de formule générale (II) pour lequel R" est un atome d'hydrogène ci-après appelé pristinamycine IIG (PIIG) sont des produits qui constituent des composantes très minoritaires des streptogramines dont la proportion en poids est inférieure à 0,5 % dans les lots de produit naturel.

La cocristallisation s'effectue dans la stoechiométrie constante de 1 mole de composante(s) de formule générale (I) avec 2 moles de composante(s) du groupe A de formule générale (II) [cette stoechiométrie correspondant à la proportion relative d'environ 43-49/57-51 en poids].

Selon l'invention les associations cocristallisées sont préparées de la manière suivante : un mélange brut contenant au moins 30 % d'une composante minoritaire du groupe A répondant à la formule générale (II) mis en solution dans un solvant organique tel qu'une cétone (acétone, méthyléthylcétone, méthylisobutylcétone par exemple), un ester (acétate d'éthyle, acétate d'isopropyle, acétate de butyle, acétate d'isobutyle par exemple), un solvant chloré (chlorure de méthylène, chloroforme, dichloro-1,2 éthane par exemple), ou un nitrile (acétonitrile par exemple), est additionné d'une composante du groupe B définie par la formule générale (I) pour donner un composé cocristallisé dans les proportions définies ci-avant. Il est entendu que la quantité de composé de formule générale (I) introduite est choisie convenablement pour que la concentration résiduelle de ce produit (après la cocristallisation) soit inférieure à sa solubilité dans le milieu. Il est également entendu que des variations dans les teneurs respectives du milieu initial en produit de formule générale (II) et en produit de formule générale (I) n'entrainent pas de modification du composé cocristallisé obtenu.

Cette association cocristallisée présente l'avantage d'une très bonne stabilité et d'une pureté élevée.

La préparation des composantes du groupe B des streptogramines définies par la formule générale (I) peut être effectuée de la manière suivante :

Lorsque R₃ représente un radical de formule générale (III) pour lequel R et R' sont définis comme en 1), si R' est un atome de chlore ou de brome les produits de formule générale (I) peuvent être obtenus par action du dérivé N-halogéno succinimide correspondant sur la pristinamycine I pour laquelle R' est un atome d'hydrogène.

La réaction s'effectue au moyen de N-chloro ou de N-bromo succinimide dans un solvant organique comme par exemple un solvant chloré (dichlorométhane, dichloréthane, chloroforme) ou un nitrile (acétonitrile), à une température comprise entre 20 et 80°C.

Lorsque R₃ représente un radical de formule générale (III) pour lequel R et R' sont définis comme en 1), si R' est un radical alcényle contenant 3 à 5 atomes de carbone les produits de formule générale (I) peuvent être obtenus par réarrangement en milieu légèrement basique d'un sel dérivé de 4-N-alcénylammonio pristinamycine IA de formule générale : 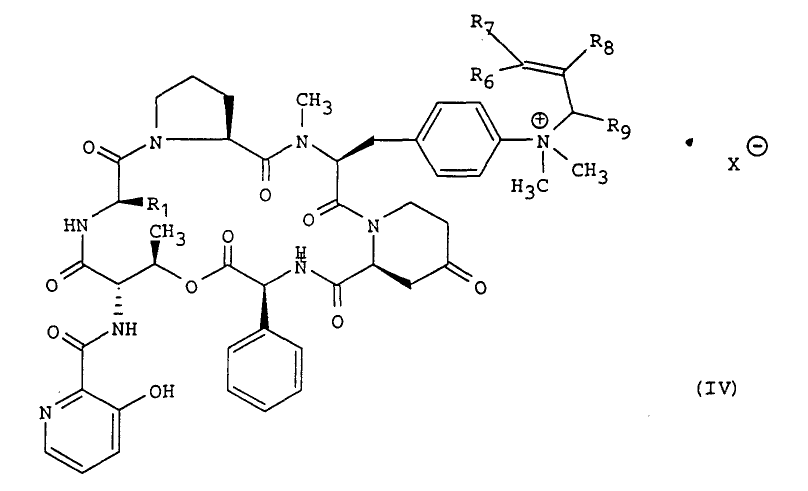 dans laquelle R₁ est défini comme ci-dessus, R₆, R₇, R₈ et R₉ sont un atome d'hydrogène ou un radical méthyle, pourvu que 2 d'entre eux au moins soient des atomes d'hydrogène et X^{⊖} représente un anion, pour donner le dérivé de formule générale : 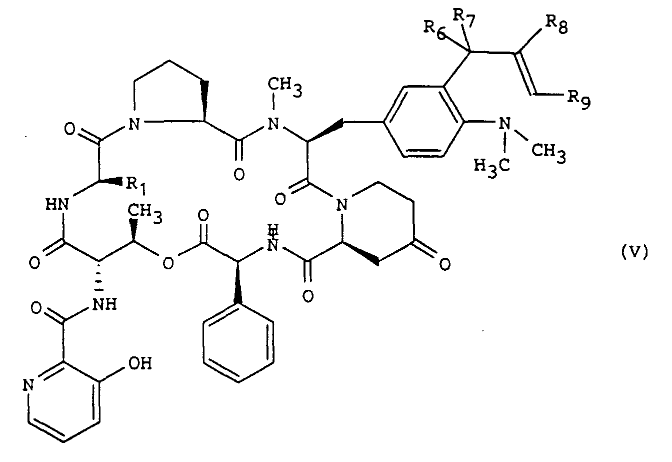 pour lequel R₁, R₆, R₇, R₈ et R₉ sont définis comme ci-dessus.

La réaction s'effectue par chauffage à une température comprise entre 80 et 100°C en milieu aqueux ou biphasique (par exemple en milieu acétate d'éthyle/eau), en présence d'acétate de sodium ou de bicarbonate de sodium ou de potassium. On utilise avantageusement un halogénure de 4-N-alcénylammonio pristinamycine IA.

L'halogénure de 4-N-alcénylammonio pristinamycine IA peut être obtenu par action d'un halogénure d'alcényle de formule générale : 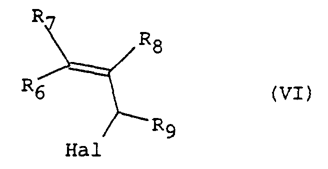 pour lequel R₆, R₇, R₈ et R₉ sont définis comme ci-dessus, et Hal représente un atome d'halogène, sur un dérivé de la pristinamycine de formule générale : 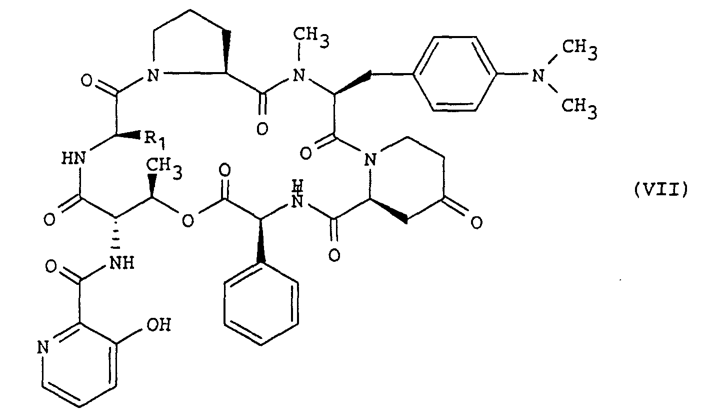 dans laquelle R₁ est défini comme précédemment.

La réaction s'effectue avantageusement dans un solvant organique tel qu'un solvant chloré (dichlorométhane, dichloréthane, chloroforme par exemple) ou un alcool (éthanol par exemple) ou dans un mélange, à une température comprise entre 20°C et la température de reflux du mélange réactionnel. De préférence on fait agir un produit de formule générale (VI) pour lequel Hal est un atome de chlore ou de brome.

Lorsque R₃ représente un radical de formule générale (III) pour lequel R et R' sont définis comme en 2), les produits de formule générale (I) peuvent être obtenus comme décrit ci-après dans les exemples, à partir d'une souche d'un microorganisme producteur de streptogramines possèdant au moins une modification génétique affectant la biosynthèse d'un précurseur des streptogramines du groupe B, ladite souche mutante cultivée dans un milieu de culture adéquat, complémenté avec au moins un précurseur original autre que celui dont la biosynthèse est altérée (et correspondant à la structure souhaitée), produit les dérivés de streptogramines attendus.

Les souches mises en oeuvre dans le cadre de la présente invention sont donc des souches productrices de streptogramines naturelles (pristinamycine IA, pristinamycine IB,. virginiamycine S), mutées. La/lesdites modifications génétiques peuvent être localisées soit au niveau d'un des gènes impliqués dans la biosynthèse desdits précurseurs soit en dehors de la région codante, par exemple dans les régions responsables de l'expression et/ou de la régulation transcriptionnelle ou post-transcriptionnelle desdits gènes ou dans une région appartenant au transcript contenant lesdit gènes.

Notamment, les souches mutantes possèdent une ou plusieurs modifications génétiques au niveau d'au moins un de leurs gènes impliqués dans la biosynthèse des précurseurs des streptogramines du groupe B. Cette ou ces modifications génétiques altèrent l'expression dudit gène c'est à dire rendent ce gène, et le cas échéant un autre des gènes impliqués dans la biosynthèse des précurseurs, partiellement ou totalement incapable de coder pour l'enzyme naturelle impliquée dans la biosynthèse d'au moins un précurseur.

Les gènes, susceptibles d'être mutés sont de préférence les gènes impliqués dans la biosynthèse du précurseur 4-diméthylamino-L-phénylalanine (DMPAPA). Il s'agit plus préférentiellement des gènes papA, papM, papB (SEQ ID N°3), papC (SEQ ID N°2). Les gènes papA et papM ont déjà été décrits dans la demande de brevet PCT/FR93/0923.

En complémentant le milieu de culture de souches mutantes selon l'invention, avec au moins un précurseur original, il s'avère possible d'orienter la biosynthèse vers les nouvelles streptogramines de formule générale (I).

Les précurseurs mis en oeuvre dans le cadre de la présente invention sont des dérivés de la phénylalanine ainsi que des dérivés d'acides α-cétocarboxyliques qui peuvent être représentés par la formule générale: 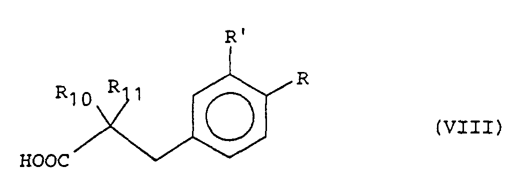 dans laquelle R et R' sont définis comme précédemment en 2) et R₁₀ est un atome d'hydrogène et R₁₁ est un radical amino, ou bien R₁₀ et R₁₁ forment ensemble un radical oxo.

A titre de précurseurs convenant à l'invention, on peut notamment citer les suivants:

4-diéthylaminophénylalanine, 4-éthylaminophénylalanine, 4-méthylthiophénylalanine, 4-méthylphénylalanine, 4-méthoxyphénylalanine, 4-trifluorométhoxyphénylalanine, 4-chlorophénylalanine, 4-bromophénylalanine, 4-iodophénylalanine, 4-trifluorométhylphénylalanine, 4-tert-butylphénylalanine, 4-isopropylphénylalanine, 3-méthylaminophénylalanine, 3-méthoxyphénylalanine, 3-méthylthiophénylalanine, 3-fluorophénylalanine, acide 4-tert-butylphénylpyruvique, 4-fluorophénylalanine, 3-éthoxyphénylalanine, 3,4-diméthylphénylalanine, 3-méthylphénylalanine, 4-butylphénylalanine, 3-trifluorométhylphénylalanine et 3-éthylaminophénylalanine, 4-aminométhylphénylalanine.

La préparation et la séparation des composantes des streptogramines naturelles des groupes A [streptogramines de formule générale (II)] et B [et notamment des produits de formule générale (VII)] est effectuée par fermentation et isolement des constituants à partir du môut de fermentation selon ou par analogie avec la méthode décrite par J. Preud'homme et coll., Bull. Soc. Chim. Fr., vol.2, 585 (1968), dans Antibiot. & Chemother., 5, 632 (1955) ou 7, 606 (1957), dans Chromatog. Sym., 2° Bruxelles, 181 (1962), dans Antibiot. Ann., 728 784 (1954-55), dans le brevet US 3 299 047 ou dans Streptogramine als Modelsysteme für den Kationentransport durch Membranen, Dissertation zur Erlangung des Doktorgrades der Mathematisch-Naturwissenschaftlichen Facultät der Georg-August Universität zu Göttingen, Göttingen 1979 ou comme décrit ci-après dans les exemples. Notamment dans le cas des pristinamycines, la séparation des composantes des groupes A et B est effectuée par mise en suspension de la streptogramine brute dans un solvant organique comme un acétate (acétate d'éthyle par exemple) suivie de la filtration ou de la centrifugation de la composante brute du groupe A, et de l'extraction de la composante du groupe B en milieu acide aqueux suivie d'une réextraction en milieu chlorométhylénique. La séparation des composantes des groupes A et B peut également être effectuée par extraction acide d'une solution de streptogramine brute dans la méthylisobutylcétone, puis isolement par extraction de la composante du groupe B à partir de la phase aqueuse et isolement de la composante du groupe A par précipitation à partir de la phase organique.

Après séparation, la purification des composantes du groupe B des streptogramines peut être mise en oeuvre par cristallisation dans un alcool tel que l'éthanol, le méthanol ou l'isopropanol, dans un acétate (acétate d'isopropyle ou acétate de butyle par exemple), dans une cétone (méthyléthylcétone par exemple) ou dans l'acétonitrile ou par chromatographie. La purification des composantes du groupe A de formule générale (II) peut être effectuée par chromatographie en éluant par un mélange acétonitrile-eau.

Alternativement la préparation des composantes naturelles des groupes A et B respectivement de formule générale (II) et notamment de formule générale (VII) est effectuée comme décrit dans la demande de brevet français 2 689 518, par fermentation séparée.

Ainsi, selon l'invention il est maintenant possible d'obtenir une nouvelle forme purifiée et cristallisée de streptogramine dont le taux d'impuretés, la définition et la constance de la composition sont améliorés et qui possède de surcroît une activité in vivo améliorée ainsi qu'une faible toxicité. La nouvelle association cocristallisée pourra ainsi pallier à l'absence de traitement par un antibactérien de cette classe dans de nombreux pays.

Les associations selon l'invention manifestent une action biologique in vivo supérieure à celle des associations de streptogramines, jusqu'à présent connues, sous forme de mélange de poudres.

La nouvelle association cocristallisée d'une composante du groupe B des streptogramines de formule générale (I) et d'une composante du groupe A des streptogramines de formule générale (II) présente une activité in vivo particulièrement intéressante notamment sur les germes à Gram-positif. In vivo, chez la souris elle s'est montrée active sur Staphylococcus aureus IP 8203 à des doses de 25 à 50 mg/kg par voie orale.

De plus la nouvelle association ne présente pas de toxicité : aucun signe de toxicité ne se manifeste chez la souris à la dose de 150 mg/kg par voie orale (2 administrations).

L'association cocristallisée selon l'invention peut être également utilisée comme moyen de purification d'une composante minoritaire des streptogramines répondant à la formule générale (II).

En effet, il n'a jamais été possible de purifier une composante du groupe A de formule générale (II) par cristallisation.

Il a maintenant été montré que la composante du groupe A de formule générale (II) peut être obtenue à l'état pur en passant par l'intermédiaire de l'association cocristallisée définie ci-dessus.

Selon l'invention, lorsque l'association cocristallisée est utilisée comme moyen de purification de la composante de formule générale (II), celle-ci peut être obtenue par extraction acide d'une solution du composé cocristallisé dans une cétone (méthylisobutylcétone par exemple), puis isolement de la composante du groupe A par précipitation à partir de la phase organique, lorsqueR ou R' représentent alcoylamino ou dialcoylamino ; ou alternativement par chromatographie liquide haute performance.

D'un intérêt particulier sont les associations cocristallisées qui comprennent au moins une composante du groupe B des streptogramines définie par la formule générale (I), dans laquelle les symboles R₁ et R₂ sont définis comme précedemment, et le symbole R₃ représente un radical benzyle substitué de formule générale (III) dans laquelle
1) à condition que R₂ soit simultanément hydrogène, R représente NR₄R₅ pour lequel les symboles R₄ et R₅ sont l'un un atome d'hydrogène ou un radical méthyle et l'autre un radical méthyle et R' est un atome de chlore ou de brome, ou représente un radical alcényle contenant 3 à 5 atomes de carbone si R₄ et R₅ sont des radicaux méthyle, ou bien
2) R est un atome d'hydrogène et R' représente un radical alcoylamino ou dialcoylamino, le reste d'un éther-oxyde ou un radical alcoylthio, ou bien
   R est un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical alcényl alcoyl amino dont la partie alcényle contient 3 ou 4 atomes de carbone et la partie alcoyle est définie comme ci-dessus, le reste d'un éther-oxyde, un radical alcoylthio, alcoyle contennant 1 à 6 atomes de carbone en chaîne droite ou ramifiée ou trihalogénométhyle, et R' est un atome d'hydrogène, ou bien
   R est un radical alcoyle contenant 1 à 6 atomes de carbone en chaîne droite ou ramifiée et R' représente un atome d'halogène, cocristallisée avec au moins une composante "minoritaire" du groupe A des streptogramines telle que définie précédemment, étant entendu que, sauf mention spéciale, les radicaux alcoyle sont droits ou ramifiés et contiennent 1 à 4 atomes de carbone.

Et parmi ces produits, les associations préférées sont les associations cocristallisées qui comprennent au moins une composante du groupe B des streptogramines définie par la formule générale (I), dans laquelle le symbole R₁ est un radical éthyle, le symbole R₂ est un atome d'hydrogène, et
le symbole R₃ représente un radical benzyle substitué de formule générale (III) dans laquelle :
1) à condition que R₂ soit simultanément hydrogène, R représente NR₄R₅ pour lequel les symboles R₄ et R₅ sont l'un un atome d'hydrogène ou un radical méthyle et l'autre un radical méthyle et R' est un atome de chlore ou de brome, ou représente un radical allyle si R₄ et R₅ sont des radicaux méthyle, ou bien
2) R est un atome d'hydrogène et R' représente un radical alcoylamino ou dialcoylamino, un radical méthoxy, éthoxy, allyloxy ou trifluorométhoxy, ou un radical alcoylthio, ou bien
   R est un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical allyl alcoyl amino dont la partie alcoyle est définie comme ci-dessus, un radical méthoxy, éthoxy, allyloxy ou trifluorométhoxy, un radical alcoylthio, alcoyle contennant 1 à 6 atomes de carbone en chaîne droite ou ramifiée ou trifluorométhyle, et R' est un atome d'hydrogène, ou bien
   R est un radical alcoyle contenant 1 à 6 atomes de carbone en chaîne droite ou ramifiée et R' représente un atome d'halogène, cocristallisée avec au moins une composante "minoritaire" du groupe A des streptogramines telle que définie précédemment, étant entendu que, sauf mention spéciale, les radicaux alcoyle sont droits ou ramifiés et contiennent 1 à 4 atomes de carbone.

Les exemples suivants donnés à titre non limitatif illustrent la présente invention.

Dans les exemples qui suivent il est entendu que les titres sont donnés en % en poids.Les chromatographies analytiques (CLHP) ont été réalisées en mode isocratique à 0,8 ml/mn avec un éluant composé de 40 % d'acétonitrile et de 60 % de tampon phosphate 0,1M pH=2,9 sur une colonne de Nucléosil 100 C8® en 5µ (4x133 mm, Macherey Nagel). Les streptogramines sont détectées et dosées d'après leur absorbance U.V. à 206 nm. Les titres présentés ont été normalisés à 100 %. Les diagrammes de diffraction X sont effectués sur diffractomètre Phillips PW1700® à anticathode de cobalt. La référence 100 est donnée pour la raie à 15,8 Å. Les valeurs relatives sont estimées par la mesure de la hauteur de la raie, fond continu déduit. Dans les exemples qui suivent, le spectre de diffraction X du produit cocristallisé est différent du spectre de la composante du groupe B cristallisée seule dans le même solvant, lorsqu'elle existe sous forme cristallisée.

### PREPARATION D'UN PRODUIT COCRISTALLISE :

### Exemple 1

A une solution de 784 mg de pristinamycine IIB dans 7 cm³ d'acétone, on ajoute 672 mg de 4ε-bromo pristinamycine I_{A}. Après tiédissement pour faciliter le passage en solution puis retour à 20°C, on observe une précipitation. Après 20 heures d'agitation à 20°C, le produit est filtré, lavé avec de l'acétone, séché à poids constant sous pression réduite (135 Pa). On obtient ainsi 940 mg de l'association cocristallisée de 2 molécules de pristinamycine IIB et de une molécule de 4ε-bromo pristinamycine I_{A} sous forme de cristaux blancs fondant à 188°C, titrant environ 53 % en pristinamycine IIB et 44 % en 4ε-bromo pristinamycine I_{A}.

Diagramme de diffraction X :

Dans le tableau ci-après sont données les intensités relatives des raies principales.

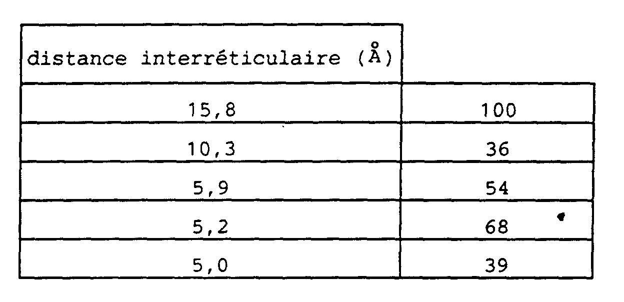

### Exemple 2

En opérant comme décrit ci-dessus à l'exemple 1 mais en engageant 112 mg de pristinamycine IIB et 96 mg de 4ε-chloro pristinamycine IA dans 1 cm³ d'acétone, on obtient ainsi 130 mg de l'association cocristallisée de 2 molécules de pristinamycine IIB et de une molécule de 4ε-chloro pristinamycine I_{A} sous forme de cristaux blancs fondant à 190°C, titrant environ 55 % en pristinamycine IIB et 45 % en 4ε-chloro pristinamycine I_{A}.

Diagramme de diffraction X :

Dans le tableau ci-après sont données les intensités relatives des raies principales.

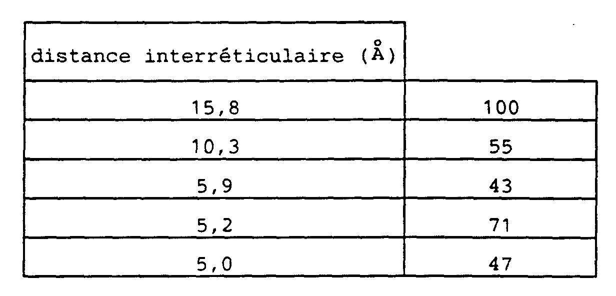

### Exemple 3

En opérant comme décrit ci-dessus à l'exemple 1 mais en engageant 112 mg de pristinamycine IIB et 96 mg de 4ε-allyl pristinamycine I_{A} dans 1 cm³ d'acétone, on obtient 160 mg de l'association cocristallisée de 2 molécules de pristinamycine IIB et de une molécule de 4ε-allyl pristinamycine I_{A} sous forme de cristaux blancs fondant à 182°C, titrant environ 53 % en pristinamycine IIB et 47 % en 4ε-allyl pristinamycine I_{A}.

Diagramme de diffraction X :

Dans le tableau ci-après sont données les intensités relatives des raies principales.

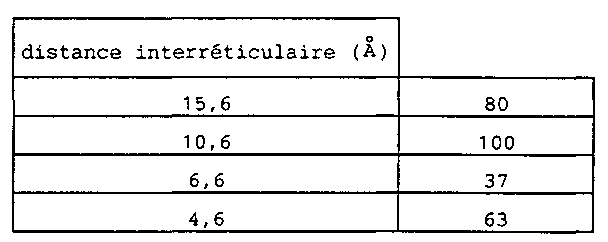

### Exemple 4

En opérant comme décrit ci-dessus à l'exemple 1 mais en engageant 560 mg de pristinamycine IIB et 480 mg de 4ε-bromo pristinamycine I_{B} dans 5 cm³ d'acétone, on obtient 730 mg de l'association cocristallisée de 2 molécules de pristinamycine IIB et de une molécule de 4ε-bromo pristinamycine I_{B} sous forme de cristaux blancs fondant à 179°C titrant environ 53 % en pristinamycine IIB et 47 % en 4ε-bromo pristinamycine I_{B}.

Diagramme de diffraction X :

Dans le tableau ci-après sont données les intensités relatives des raies principales.

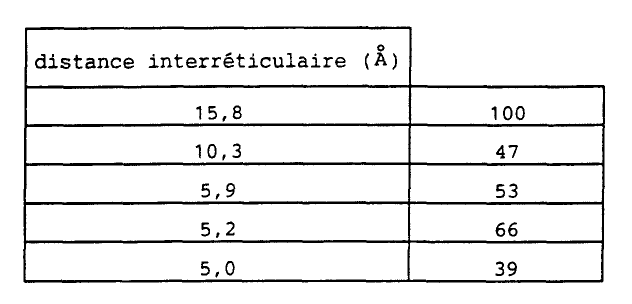

### Exemple 5

En opérant comme décrit ci-dessus à l'exemple 1 mais en engageant 784 mg de pristinamycine IIB et 672 mg de 4ε-chloro pristinamycine I_{B} dans 7 cm³ d'acétone, on obtient 1,0 g de l'association cocristallisée de 2 molécules de pristinamycine IIB et de une molécule de 4ε-chloro pristinamycine I_{B} sous forme de cristaux blancs fondant à 178°C titrant environ 57 % en pristinamycine IIB et 43 % en 4ε-chloro pristinamycine I_{B}.

Diagramme de diffraction X :

Dans le tableau ci-après sont données les intensités relatives des raies principales.

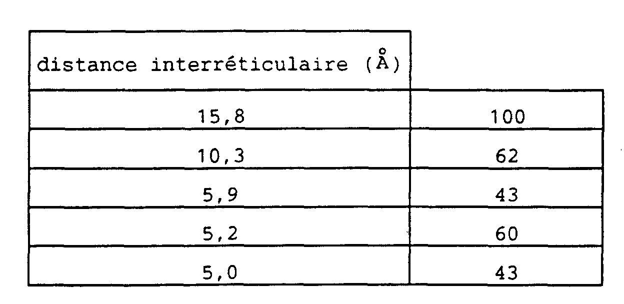

### Exemple 6

En opérant comme décrit ci-dessus à l'exemple 1 mais en engageant 425 mg de pristinamycine IIB et 364 mg de dés(4N-diméthylamino) 4ζ-tertiobutyl pristinamycine IA dans 3,8 cm³ d'acétone, on obtient 305 mg de l'association cocristallisée de 2 molécules de pristinamycine IIB et de une molécule de dés(4ζ-diméthylamino) 4ζ-tertiobutyl pristinamycine I_{A} sous forme de cristaux blancs fondant à 170°C, titrant environ 57 % en pristinamycine IIB et 43 % en dés(4ζ-diméthylamino) 4ζ-tertiobutyl pristinamycine I_{A}.

Diagramme de diffraction X :

Dans le tableau ci-après sont données les intensités relatives des raies principales.

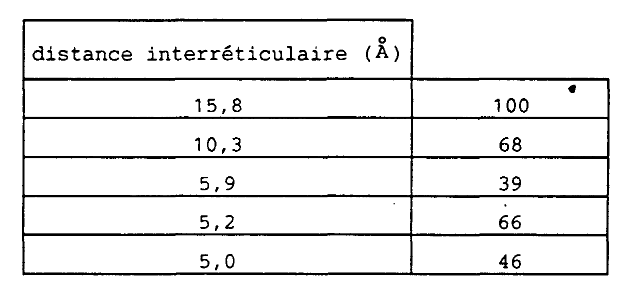

### Exemple 7

En opérant comme décrit ci-dessus à l'exemple 1 mais en engageant 168 mg de pristinamycine IIB et 144 mg de dés(4ζ-diméthylamino) 4ζ-isopropyl pristinamycine IA dans 1,5 cm³ d'acétone, on obtient 270 mg de l'association cocristallisée de 2 molécules de pristinamycine IIB et de une molécule de dés(4ζ-diméthylamino) 4ζ-isopropyl pristinamycine I_{A} sous forme de cristaux blancs fondant à 180°C, titrant environ 56 % en pristinamycine IIB et 44 % en dés(4ζ-diméthylamino) 4ζ-isopropyl pristinamycine I_{A}.

Diagramme de diffraction X :

Dans le tableau ci-après sont données les intensités relatives des raies principales.

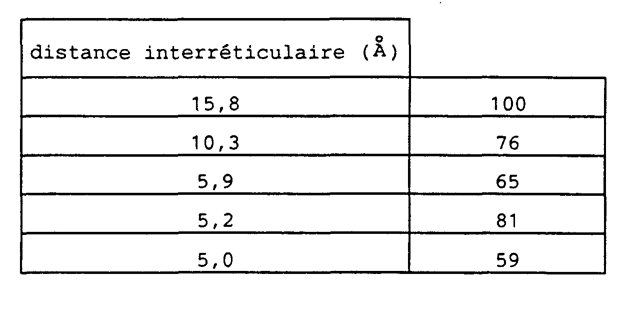

### Exemple 8

En opérant comme décrit ci-dessus à l'exemple 1 mais en engageant 224 mg de pristinamycine IIB et 192 mg de dés(4N-diméthylamino) 4ζ-méthoxy pristinamycine I_{A} dans 2 cm³ d'acétone, on obtient 320 mg de l'association cocristallisée de 2 molécules de pristinamycine IIB et de une molécule de dés(4ζ-diméthylamino) 4ζ-méthoxy pristinamycine I_{A} sous forme de cristaux blancs fondant à 169°C, titrant environ 54 % en pristinamycine IIB et 46 % en dés(4ζ-diméthylamino) 4ζ-méthoxy pristinamycine I_{A}.

Diagramme de diffraction X :

Dans le tableau ci-après sont données les intensités relatives des raies principales.

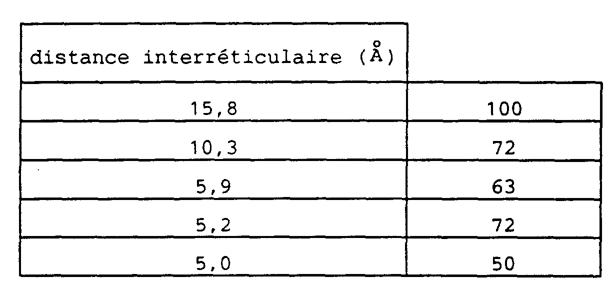

### Exemple 9

En opérant comme décrit ci-dessus à l'exemple 1 mais en engageant 149 mg de pristinamycine IIB et 128 mg de dés (4M-diméthylamino) 4ζ-méthyl pristinamycine I_{A} dans 1,3 cm³ d'acétone, on obtient 205 mg de l'association cocristallisée de 2 molécules de pristinamycine IIB et de une molécule de dés(4ζ-diméthylamino) 4ζ-méthyl pristinamycine I_{A} sous forme de cristaux blancs, titrant environ 56 % en pristinamycine IIB et 44 % en dés(4ζ-diméthylamino) 4ζ-méthyl pristinamycine I_{A}.

### Exemple 10

A une solution de 1,94 g de pristinamycine IIB dans 6 cm³ d'acétone on ajoute une solution de 1,6 g dés(4ζ-diméthylamino) 4ζ-méthythio pristinamycine I_{A} dans 7 cm³ d'acétone. Le mélange est agité 30 minutes puis filtré. Les cristaux obtenus sont rincés par 5 fois 1 cm³ d'acétone puis par 3 fois 10 cm³ de pentane puis séché à poids constant sous pression réduite (2,7 kPa). On obtient ainsi 2,3 g de l'association cocristallisée de 2 molécules de pristinamycine IIB et de une molécule de dés(4ζ-diméthylamino) 4ζ-méthythio pristinamycine I_{A} sous forme de cristaux blancs fondant à 183°C, titrant environ 51 % en pristinamycine IIB et 49 % en dés(4ζ-diméthylamino) 4ζ-méthythio pristinamycine I_{A}.

Diagramme de diffraction X :

Dans le tableau ci-après sont données les intensités relatives des raies principales.

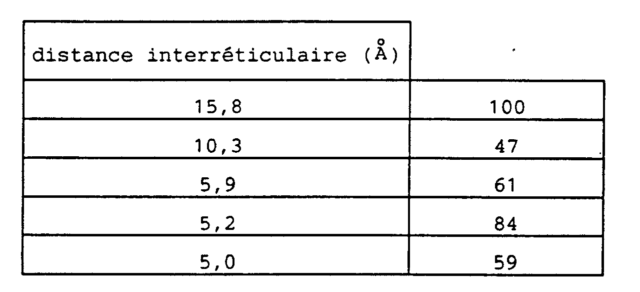

### Exemple 11

En opérant comme à l'exemple 10 mais à partir de 106 mg de pristinamycine IIB et de 86 mg de dés(4ζ-diméthylamino) 4ε-méthylamino pristinamycine I_{A} et après 18 heures d'agitation, on obtient 41 mg de l'association cocristallisée de 2 molécules de pristinamycine IIB et de une molécule de dés(4ζ-diméthylamino) 4ε-méthylamino pristinamycine IA sous forme de cristaux blancs titrant environ 53 % en pristinamycine IIB et 47 % en dés(4ζ-diméthylamino) 4ε-méthylamino pristinamycine I_{A}

Diagramme de diffraction X :

Dans le tableau ci-après sont données les intensités relatives des raies principales.

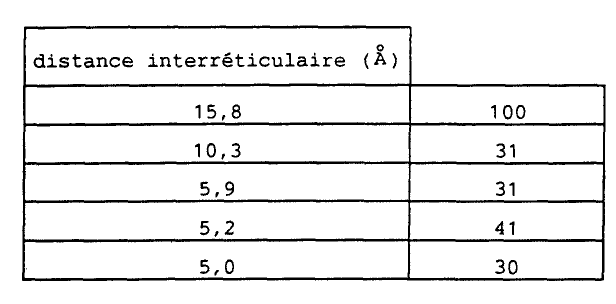

### Exemple 12

En opérant comme à l'exemple 10 mais à partir de 57 mg de pristinamycine IIB et de 48 mg de dés(4ζ-diméthylamino) 4ζ-trifluorométhyl pristinamycine I_{A} et après 17 heures d'agitation, on obtient 62 mg de l'association cocristallisée de 2 molécules de pristinamycine IIB et de une molécule de dés(4ζ-diméthylamino) 4ζ-trifluorométhyl pristinamycine I_{A} sous forme de cristaux blancs fondant à 183°C, titrant environ 55 % en pristinamycine IIB et 45 % en dés(4ζ-diméthylamino) 4ζ-trifluorométhyl pristinamycine I_{A}.

### Exemple 13

En opérant comme à l'exemple 10 mais à partir de 370 mg de pristinamycine IIB et de 300 mg de dés(4ζ-diméthylamino) 4ε-méthoxy pristinamycine I_{A} et après 17 heures d'agitation, on obtient 330 mg de l'association cocristallisée de 2 molécules de pristinamycine IIB et de une molécule de dés(4ζ-diméthylamino) 4ε-méthoxy pristinamycine I_{A} sous forme de cristaux blancs fondant à 180°C, titrant environ 48 % en pristinamycine IIB et 52 % en dés(4ζ-diméthylamino) 4ε-méthoxy pristinamycine I_{A}.

Diagramme de diffraction X :

Dans le tableau ci-après sont données les intensités relatives des raies principales.

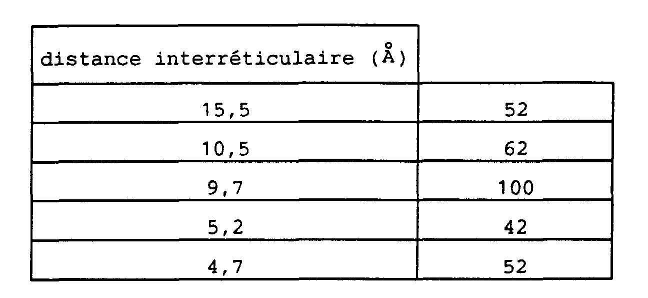

### Exemple 14

En opérant comme à l'exemple 10 mais à partir de 70 mg de pristinamycine IIB et de 75 mg de dés(4ζ-diméthylamino) 4ε-fluoro 4ζ-méthyl pristinamycine I_{A} (titrant 75 %) et après 3 heures 45 minutes d'agitation, on obtient 57 mg de l'association cocristallisée de 2 molécules de pristinamycine IIB et de une molécule de dés(4ζ-diméthylamino) 4ε-fluoro 4ζ-méthyl pristinamycine I_{A} sous forme de cristaux blancs fondant à 184°C, titrant environ 53 % en pristinamycine IIB et 47 % en dés(4ζ-diméthylamino) 4ε-fluoro 4ζ-méthyl pristinamycine I_{A}.

Diagramme de diffraction X :

Dans le tableau ci-après sont données les intensités relatives des raies principales.

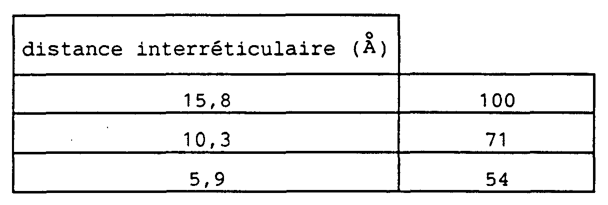

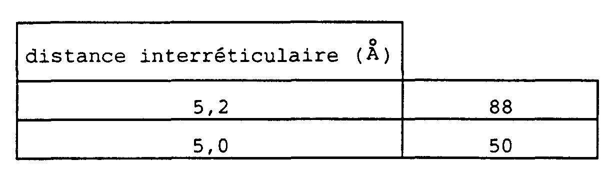

### Exemple 15

En opérant comme décrit ci-dessus à l'exemple 1 mais en engageant 61 mg de pristinamycine IIB et 50 mg de 4ε-éthoxy-dés(4ζ-diméthylamino) pristinamycine I_{A} dans 0,6 cm³ d'acétone, on obtient 30 mg de l'association cocristallisée de 2 molécules de pristinamycine IIB et de une molécule de 4ε-éthoxy-dés(4ζ-diméthylamino) pristinamycine I_{A} sous forme de cristaux blancs fondant à 182°C, titrant 59 % en pristinamycine IIB et 41 % en 4ε-éthoxy-dés(4ζ-diméthylamino) pristinamycine I_{A}.

Diagrammme de diffraction X :

Dans le tableau ci-dessous sont données les intensités relatives des raies principales.

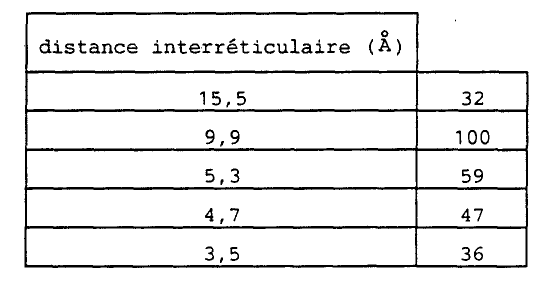

### Exemple 16

En opérant comme décrit ci-dessus à l'exemple 1 mais en engageant 97 mg de pristinamycine IIB et 80 mg de 4ζ-éthoxy-dés(4ζ-diméthylamino) pristinamycine I_{A} dans 0,7 cm³ d'acétone, on obtient 100 mg de l'association cocristallisée de 2 molécules de pristinamycine IIB et d'une molécule de 4ζ-éthoxy-dés(4ζ-diméthylamino) pristinamycine I_{A} sous forme de cristaux blancs fondant à 196°C, titrant 54,6 % en pristinamycine IIB et 45,4 % en 4ζ-éthoxy-dés(4ζ-diméthylamino) pristinamycine I_{A}.

### Exemple 17

En opérant comme décrit ci-dessus à l'exemple 1 mais en engageant 56 mg de pristinamycine IIB et 47 mg de 4ζ-allyloxy-dés(4ζ-diméthylamino) pristinamycine I_{A} dans 0,5 cm³ d'acétone, on obtient ainsi 67 mg de l'association cocristallisée de 2 molécules de pristinamycine IIB et de une molécule de 4ζ-allyloxy-dés(4ζ-diméthylamino) pristinamycine I_{A} sous forme de cristaux blancs fondant à 196°C, titrant 57,1 % en pristinamycine IIB et 42,9% en 4ζ-allyloxy-dés(4ζ-diméthylamino) pristinamycine I_{A}.

### Exemple 18

En opérant comme décrit ci-dessus à l'exemple 1 mais en engageant 62 mg de pristinamycine IIB et 53 mg de 4ζ-éthylamino-dés(4ζ-diméthylamino) pristinamycine I_{A} dans 0,6 cm³ d'acétone, on obtient 47 mg de l'association cocristallisée de 2 molécules de pristinamycine IIB et de une molécule de 4ζ-éthylamino-dés(4ζ-diméthylamino) pristinamycine I_{A} sous forme de cristaux blancs fondant à 188°C, titrant 55,7 % en pristinamycine IIB et 44,3 % en 4ζ-éthylamino-dés(4ζ-diméthylamino) pristinamycine I_{A}.

### Exemple 19

En opérant comme décrit ci-dessus à l'exemple 1 mais en engageant 178 mg de pristinamycine IIB et 153 mg de 4ζ-trifluorométhoxy-dés(4ζ-diméthylamino) pristinamycine I_{A} dans 1,8 cm³ d'acétone, on obtient 188 mg de l'association cocristallisée de 2 molécules de pristinamycine IIB et de une molécule de 4ζ-trifluorométhoxy-dés(4ζ-diméthylamino) pristinamycine I_{A} sous forme de cristaux blancs fondant à 184°C, titrant 54,1 % en pristinamycine IIB et 45,9 % en 4ζ-trifluorométhoxy-dés(4ζ-diméthylamino) pristinamycine I_{A}.

Diagramme de diffraction X :

Dans le tableau ci-dessous sont données les intensités relatives des raies principales.

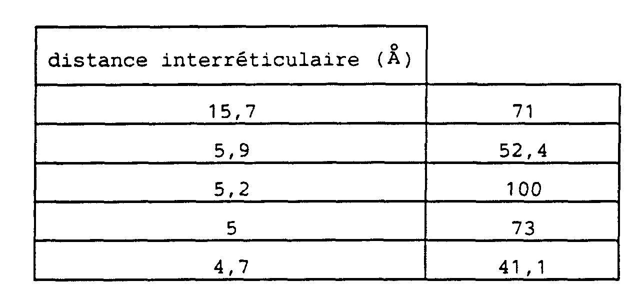

### Exemple 20

En opérant comme décrit ci-dessus à l'exemple 1 mais en engageant 61 mg de pristinamycine IIB et 50 mg de 4ε-méthylthio-dés(4ζ-diméthylamino) pristinamycine I_{A} dans 0,6 cm³ d'acétone, on obtient ainsi 38 mg de l'association cocristallisée de 2 molécules de pristinamycine IIB et de une molécule de 4ε-méthylthio-dés(4ζ-diméthylamino) pristinamycine I_{A} sous forme de cristaux blancs fondant à 188°C, titrant 53,1 % en pristinamycine IIB et 46,9 % en 4ε-méthylthio-dés(4ζ-diméthylamino) pristinamycine I_{A}.

Diagramme de diffraction X :

Dans le tableau ci-dessous sont données les intensités relatives des raies principales.

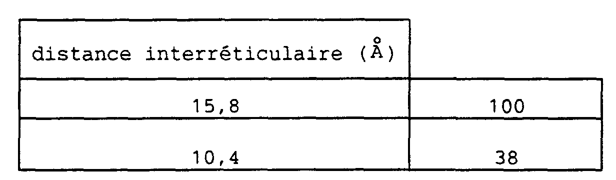

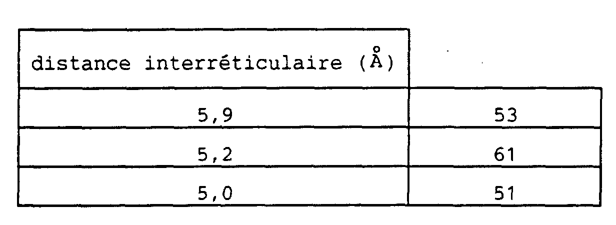

### Exemple 21

En opérant comme décrit ci-dessus à l'exemple 1 mais en engageant 35 mg de pristinamycine IIB et 30 mg de 4ζ-(allyl éthyl amino) dés(4ζ-diméthylamino) pristinamycine I_{A} dans 0,3 cm³ d'acétone, on obtient ainsi 30 mg de l'association cocristallisée de 2 molécules de pristinamycine IIB et de une molécule de 4ζ-(allyl éthyl amino) dés(4ζ-diméthylamino) pristinamycine I_{A} sous forme de cristaux blancs fondant à 174°C, titrant 52,3 % en pristinamycine IIB et 47,7 % en 4ζ-(allyl éthyl amino) dés(4ζ-diméthylamino) pristinamycine I_{A}.

### Exemple 22

En opérant comme décrit ci-dessus à l'exemple 1 mais en engageant 42 mg de pristinamycine IIB et 36 mg de 4ζ-(éthyl propyl amino) dés(4ζ-diméthylamino) pristinamycine I_{A} dans 0,5 cm³ d'acétone, on obtient 16 mg de l'association cocristallisée de 2 molécules de pristinamycine IIB et de une molécule de 4ζ-(éthyl propyl amino) dés(4ζ-diméthylamino) pristinamycine I_{A} sous forme de cristaux blancs fondant à 193°C, titrant 51 % en pristinamycine IIB et 49 % en 4ζ-(éthyl propyl amino) dés(4ζ-diméthylamino) pristinamycine I_{A}.

### PREPARATION DES COMPOSANTES DU GROUPE B :

### Exemple A

### 4ε-chloro pristinamycine I_{A}.

On place dans un ballon 8 g de pristinamycine IA dans 80 cm³ d'acétonitrile puis on ajoute 1,39 g de N-chlorosuccinimide. Le mélange est chauffé au reflux pendant 16 heures 30 minutes puis on ajoute 0,12 g de N-chlorosuccinimide et on poursuit le reflux 3 heures. Le mélange réactionnel est concentré à sec sous pression réduite (2,7 kPa) à 30°C. Le solide obtenu est repris par 50 cm³ de dichlorométhane et 60 cm³ d'eau distillée additionnée de chlorure de sodium, la phase aqueuse est décantée puis la phase organique lavée par 50 cm³ d'eau distillée saturée en chlorure de sodium. La phase organique est décantée, séchée sur sulfate de magnésium, filtrée puis concentrée à sec sous pression réduite (2,7 kPa) à 30°C pour donner un solide jaune qui est recristallisé dans 100 cm³ de propanol-1 au reflux puis une deuxième fois dans 50 cm³ de propanol-1 au reflux. Après refroidissement, filtration des cristaux et séchage sous pression réduite (135 Pa) à 50°C on obtient 3g de 4ε-chloro pristinamycine I_{A} sous forme de cristaux beige clair fondant à 220°C.

Spectre de R.M.N. du proton (300 MHz, CDCl₃, δ en ppm): 0,58 (dd, J=16 et 6 Hz, 1H, 5 β₂), 0,91 (t, J=7,5 Hz, 3H: CH₃ 2 γ), de 1,05 à 1,35 (mt, 2H: 3 β₂ et 3 γ₂), 1,32 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,17 (mt, 1H, 5 δ₂), 2,39 (d large, J=16 Hz, 1H: 5 δ₁), 2,44 (d, J=16 Hz, 1H: 5 β₁), 2,77 (s, 6H: N(CH₃ )₂ 4), 2,85 (dt, J=13,5 et 4,5 Hz, 1H: 5 ε₂), 2,97 (dd, J=12 et 5 Hz, 1H: 4 β₂), 3,23 (s, 3H: NCH₃ 4), 3,35 (t, J=12 Hz, 1H: 4 β₁), 3,30 et 3,58 (2 mts, 1H chacun: CH₂ 3 δ), 4,57 (dd, J=8 et 7,5 Hz, 1H, 3 α), 4,76 (dd large, J=13,5 et 8 Hz, 1H: 5 ε₁), 4,85 (mt, 1H: 2α), 4,90 (dd, J=10 et 1,5 Hz, 1H: 1α), 5,25 (dd, J=12 et 5 Hz, 1H: 4 α), 5,31 (d large, J=6 Hz, 1H: 5 α), 5,86 (d, J=9,5 Hz, 1H: 6 α), 5,90 (mt, 1H: 1β), 6,50 (d, J=10 Hz, 1H: NH 2), 6,97 (d, J=8 Hz, 1H: H 5 de l'aromatique en 4), 7,08 (dd, J=8 et2 Hz, 1H: H 6 de l'aromatique en 4), de 7,15 à 7,40 (mt, 6H: H Aromatiques 6 et H 2 de l'aromatique en 4), 7,43 (dd, J=8,5 et 2 Hz, 1H: 1' H₄), 7,52 (dd, J=8,5 et 4,5 Hz, 1H: 1' H₅), 7,83 (dd, J =4,5 et 2 Hz, 1H: 1' H₆), 8,38 (d, J=10 Hz, 1H: NH 1), 8,73 (d, J=9,5 Hz, 1H: NH 6), 11,65 (s, 1H: OH).

### Exemple B

### 4ε-bromo pristinamycine I_{A}

On place dans un ballon 30 g de pristinamycine I_{A} dans 300 cm³ de dichlorométhane puis on ajoute 6,85 g de N-bromosuccinimide. Le mélange est agité à température ambiante pendant 29 heures puis concentré à sec sous pression réduite. Le solide obtenu est agité dans 400 cm³ d'éther diéthylique, filtré puis lavé par 2 fois 100 cm³ d'éther diéthylique. Après filtration le solide est trituré pendant 45 minutes dans 400 cm³ d'eau distillée, filtré puis lavé par 2 fois 150 cm³ d'eau. Le solide obtenu est séché puis recristallisé dans 1600 cm³ d'éthanol au reflux. Après refroidissement, filtration des cristaux et séchage sous pression réduite (135 Pa) à 50°C on obtient 23,2 g de 4ε-bromo pristinamycine I_{A} sous forme de cristaux blancs fondant à 220°C.

Spectre de R.M.N. du proton (300 MHz, CDCl₃, δ en ppm): 0,58 (dd, J=16 et 6 Hz, 1H, 5 β₂), 0,91 (t, J=7,5 Hz, 3H: CH₃ 2 γ), de 1,10 à 1,40 (mt, 2H: 3 β₂ et 3 γ₂), 1,32 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,19 (mt, 1H, 5 δ₂), 2,39 (d large, J=16 Hz, 1H: 5 δ₁), 2,44 (d, J=16 Hz, 1H: 5 β₁), 2,76 (s, 6H: N(CH₃ )₂ 4), 2,83 (dt, J=13,5 et 4 Hz, 1H: 5 ε₂), 2,97 (dd, J=12,5 et 4,5 Hz, 1H: 4 β₂), 3,23 (s, 3H: NCH₃ 4), 3,30 et 3,57 (2 mts, 1H chacun: CH₂ 3 δ), 3,33 (t, J=12,5 Hz, 1H: 4 β₁), 4,55 (dd, J=8 et 7,5 Hz, 1H, 3 α), 4,74 (dd large, J=13,5 et 8 Hz, 1H: 5 ε₁), 4,84 (mt, 1H: 2α), 4,92 (dd, J=10 et 2 Hz, 1H: 1α), 5,27 (dd, J=12,5 et 4,5 Hz, 1H: 4 α), 5,33 (d large, J=6 Hz, 1H: 5 α), 5,88( d, J=9,5 Hz, 1H: 6 α), 5,90 (mt, 1H: 1β), 6,53 (d, J=10 Hz, 1H: NH 2), 7,00 (d, J=8 Hz, 1H: H 5 de l'aromatique en 4), 7,12 (dd, J=8 et 2 Hz, 1H: H 6 de l'aromatique en 4), de 7,15 à 7,40 (mt, 5H: H Aromatiques 6), 7,43 (dd, J=8,5 et 2 Hz, 1H: 1' H₄), 7,46 (d, J=2 Hz, 1H: H 2 de l'aromatique en 4), 7,52 (dd, J=8,5 et 4,5 Hz, 1H: 1' H₅), 7,87 (dd, J=4,5 et 2 Hz, 1H: 1' H₆), 8,41 (d, J=10 Hz, 1H: NH 1), 8,74 (d, J=9,5 Hz, 1H: NH 6), 11,65 (s, 1H: OH).

### Exemple C

### 4ε-chloro pristinamycine I_{B}

En opérant comme à l'exemple A mais à partir de 1,7 g de pristinamycine I_{B}, de 320 mg de N-chlorosuccinimide dans 17 cm³ d'acétonitrile, on obtient après 1 heure 30 minutes de reflux puis concentration à sec du mélange réactionnel, 1,8 g d'un solide beige qui est purifié par chromatographie flash (éluant dichlorométhane-méthanol, 98/2) pour donner 1,2 g de 4ε-chloro pristinamycine I_{B} sous forme d'un solide jaune pâle fondant à 198°C.

Spectre de R.M.N. du proton (400 MHz, CDCl₃, δ en ppm): 0,79 (dd, J=16 et 5,5 Hz, 1H, 5 β₂), 0,91 (t, J=7,5 Hz, 3H: CH₃ 2 γ), 1,15 (mt, 1H: 3 β₂), de 1,25 à 1,40 (mt, 1H: 3 γ₂), 1,34 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,23 (mt, 1H, 5 δ₂), 2,40 (d large, J=16 Hz, 1H: 5 δ₁), 2,47(d, J=16 Hz, 1H: 5 β₁), 2,85 (dt, J=13 et 4 Hz, 1H: 5 ε₂), de 2,85 à 2,90 (mt, 1H: 4 β₂), 2,88 (s, 3H: ArNCH₃ 4), 3,25 (s, 3H: NCH₃ 4), 3,28 et 3,58 (2 mts, 1H chacun: CH₂ 3 δ), 3,31 (t, J=12 Hz, 1H: 4 β₁), 4,40 (mf, 1H: ArNH), 4,57 (t, J=7,5 Hz, 1H, 3 α), 4,78 (dd large, J=13 et 8 Hz, 1H: 5 ε₁), 4,84 (mt, 1H: 2α), 4,91 (d large, J=10 Hz, 1H: 1α), 5,23 (dd, J=12 et 5 Hz, 1H: 4 α), 5,36 (d large, J=5,5 Hz, 1H: 5 α), 5,89 (d, J=9,5 Hz, 1H: 6 α), 5,90 (mt, 1H: 1β), 6,51 (d, J=10 Hz, 1H: NH 2), 6,55 (d, J=8 Hz, 1H: H 5 de l'aromatique en 4), 7,0,2 (dd, J=8 et 2 Hz, 1H: H 6 de l'aromatique en 4), 7,13 (d, J=2 Hz, 1H: H 2 de l'aromatique en 4), de 7,15 à 7,40 (mt, 5H: H Aromatiques 6), 7,43 (d large, J=8,5 Hz, 1H: 1' H₄), 7,52 (dd, J=8,5 et 4,5 Hz, 1H: 1' H₅), 7,79 (d large, J =4,5 Hz, 1H: 1' H₆), 8,40 (d, J=10 Hz, 1H: NH 1), 8,75 (d, J=9,5 Hz, 1H: NH 6), 11,63 (s, 1H: OH).

### Exemple D

### 4ε-bromo pristinamycine I_{B}

En opérant comme à l'exemple B mais à partir de 2 g de pristinamycine I_{B}, de 420 mg de N-bromosuccinimide dans 30 cm³ de dichlorométhane, on obtient après 1 heure 30 minutes d'agitation à température ambiante, puis concentration à sec du mélange réactionnel, 2,1 g d'un solide beige qui est purifié par chromatographie flash (éluant dichlorométhane-méthanol, 98/2) pour donner 1,7 g de 4ε-bromo pristinamycine I_{B} sous forme d'un solide blanc fondant à 220°C.

Spectre de R.M.N. du proton (400 MHz, CDCl₃, δ en ppm): 0,80 (dd, J=16 et 5,5 Hz, 1H, 5 β₂), 0,90 (t, J=7,5 Hz, 3H: CH₃ 2 γ), 1,13 (mt, 1H: 3 β₂), de 1,20 à 1,40 (mt, 1H: 3 γ₂), 1,33 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,28 (mt, 1H, 5 δ₂), 2,40 (d large, J=16 Hz, 1H: 5 δ₁), 2,46 (d, J=16 Hz, 1H: 5 β₁), 2,85 (dt, J=13 et 5 Hz, 1H: 5 ε₂), 2,88 (d, J=5,5 Hz, 3H: ArNCH₃ 4), 2,90 (dd, J=12 et 4 Hz, 1H: 4 β₂), 3,24 (s, 3H: NCH₃ 4), 3,30 et 3,58 (2 mts, 1H chacun: CH₂ 3 δ), 3,31 (t, J=12 Hz, 1H: 4 β₁), 4,41 (q, J=5,5 Hz, 1H: ArNH), 4,57 (t, J=7,5 Hz, 1H, 3 α), 4,78 (dd large, J=13 et 8 Hz, 1H: 5 ε₁), 4,85 (mt, 1H: 2α), 4,91 (d large, J=10 Hz, 1H: 1α), 5,24 (dd, J=12 et 4 Hz, 1H: 4 α), 5,37 (d large, J=5,5 Hz, 1H: 5 α), 5,89 (d, J=9,5 Hz, 1H: 6 α), 5,90 (mt, 1H: 1β), 6,51 (d, J=10 Hz, 1H: NH 2), 6,53 (d, J=8 Hz, 1H: H 5 de l'aromatique en 4), 7,0,5 (dd, J=8 et 2 Hz, 1H: H 6 de l'aromatique en 4), de 7,15 à 7,40 (mt, 6H: H Aromatiques 6 et H 2 de l'aromatique en 4), 7,43 (d large, J=8,5 Hz, 1H: 1' H₄), 7,48 (dd, J=8,5 et 5 Hz, 1H: 1' H₅), 7,79 (d large, J =5 Hz, 1H: 1' H₆), 8,40 (d, J=10 Hz, 1H: NH 1), 8,76 (d, J=9,5 Hz, 1H: NH 6), 11,63 (s, 1H: OH).

### Exemple E

### 4ε-allyl pristinamycine IA

On place dans un tricol maintenu sous atmosphère d'azote 7,07 g d'acétate de sodium dans 100 cm³ d'eau distillée. La solution est portée au reflux puis on ajoute par une ampoule de coulée, une solution de 15,5 g de bromure de 4-N-allylammonio pristinamycine I_{A} dans 100 cm³ d'eau distillée. Après 2 heures de réaction on ajoute 1 g d'acétate de sodium et le mélange est agité 22 heures au reflux. Une nouvelle portion de 5 g d'acétate de sodium est ajoutée et la réaction poursuivie pendant 20 heures. Le précipité formé est filtré à chaud, rincé par 2 fois 50 cm³ d'eau distillée puis séché sous pression réduite (2,75 kPa) pour donner 7 g d'un solide blanc qui est purifié par chromatographie flash (éluant : toluène, acétone 70/30) pour donner 4,6 g de 4ε-allyl pristinamycine I_{A} sous forme d'un solide blanc fondant à 160°C.

Spectre de R.M.N. du proton (400 MHz, CDCl₃, δ en ppm): 0,42 (dd, J=16 et 5,5 Hz, 1H, 5 β₂), 0,92 (t, J=7,5 Hz, 3H: CH₃ 2 γ), de 1,15 à 1,40 (mt, 2H: 3 β₂ et 3 γ₂), 1,33 (d, J=7,5 Hz, 3H: CH₃ 1 γ), de 1,55 à 1,80 (mt, 3H: 3 γ₁ et CH₂ 2 β), de 2, 00 à 2,15 (mt, 2H, 3 β₁ et 5 δ₂), 2,30 (d large, J=16 Hz, 1H: 5 δ₁), 2,33 (d, J=16 Hz, 1H: 5 β₁), 2,63 (s, 6H: N(CH₃ )₂ 4), 2,76 (dt, J=13,5 et 4,5 Hz, 1H: 5 ε₂), 2,98 (dd, J=12 et 4,5 Hz, 1H: 4 β₂), de 3,20 à 3,40 (mt, 3H: 4 β₁ - 3 δ₁ et 1H du ArCH₂ allyle), 3,25 (s, 3H: NCH₃ 4), 3,48 (dd, J=16 et 6,5 Hz, 1H: l' autre H du ArCH₂ allyle), 3,56 (mt, 1H: 3 δ₂), 4,57 (dd, J=6,5 et 7,5 Hz, 1H, 3 α), 4,68 (dd large, J=13,5 et 7,5 Hz, 1H: 5 ε₁), 4,84 (mt, 1H: 2α), 4,90 (d large, J=10 Hz, 1H: 1α), de 5,00 à 5,15 (mt, 2H: =CH₂), 5,23 (d large, J=5,5 Hz, 1H: 5α), 5,28 (dd, J=12 et 4,5 Hz, 1H 4α), de 5,80 à 5,95 (mt, 3H: 6 α - 1β et CH. allyle), 6,53 (d, J=10 Hz, 1H: NH 2), 7,04 (mt, 3H: H Aromatiques en 4), de 7,15 à 7,40 (mt, 5H: H Aromatiques 6), 7,45 (dd, J=8,5 et 2 Hz, 1H: 1' H₄), 7,48 (dd, J=8,5 et 4 Hz, 1H: 1' H₅), 7,88 (dd, J =4 et 2 Hz, 1H: 1' H₆), 8,45 (d, J=10 Hz, 1H: NH 1), 8,76 (d, J=9,5 Hz, 1H: NH 6), 11,64 (s, 1H: OH).

Le bromure de 4-N-allylammonio pristinamycine I_{A} peut être préparé de la manière suivante :

On place dans un tricol maintenu sous atmosphère d'azote, 10 g de pristinamycine I_{A} en solution dans 25 cm³ de dichloro-1,2 éthane puis 2,5 cm³ de bromure d'allyle. Le mélange est chauffé 7 heures à 40°C puis agité à température ambiante pendant 14 heures. On ajoute alors sous agitation en 10 minutes, 200 cm³ de toluène et le mélange est agité 30 minutes. Le précipité formé est filtré, rincé par 50 cm³ de toluène puis séché sous pression réduite (135 Pa) à 45°C pour donner 10,5 g d'un solide qui est trituré dans 200 cm³ d'acétate d'éthyle à 40°C, puis à température ambiante pendant 1 heure. Le solide est filtré puis séché à 45°C sous pression réduite (135 Pa) pour donner 10 g de bromure de 4-N-allylammonio pristinamycine IA sous forme d'un solide blanc fondant vers 210°C.

Spectre de R.M.N. du proton (400 MHz, CDCl₃ avec ajout de quelques gouttes de CD₃ OD d4, δ en ppm): 0,75 (t, J=7,5 Hz, 3H: CH₃ 2 γ), de 1,00 à 1,35 (mt, 3H: 3 β₂ - 3 γ₂ et 5 β₂), 1,18 (d, J=7,5 Hz, 3H: CH₃ 1 γ), de 1,45 à 1,65 (mt, 3H: 3 γ₁ et CH₂ 2 β), 1,95 (mt, 1H: 3 β₁), 2,15 (mt, 1H: 5 δ₂), 2,28 (d large, J=16 Hz, 1H: 5 δ₁), 2,55 (d, J=16 Hz, 1H: 5 β₁), 2,72 (dt, J=13,5 et 4,5 Hz, 1H: 5 ε₂), 2,95 (s, 3H: NCH₃ 4), de 3,10 à 3,50 (mt, 4H: CH₂ 4 β et CH₂ 3 δ), 3,40 et 3,48 (2s, 6H en totalité: N(CH₃ )₂ 4), 4,35 (t, J=7,5 Hz, 1H, 3 α), de 4,40 à 4,60 (mt, 3H: NCH₂ allyle et 5 ε₁), 4,64 (mt, 1H: 2α), 4,93 (s large, 1H: 1α), de 5,30 à 5,75 (mt, 7H: CH₂ allyle- 5 α - 4 α - 6 α - 1β et CH allyle), 6,88 (d, J=10 Hz, 1H: NH 2), de 7,05 à 7,25 (mt, 8H: H Aromatiques 6 - 1' H₄ et 4 δ), 7,35 (dd, J=8 et 4 Hz, 1H: 1' H₅), 7,60 (d, J=8,5 Hz, 2H: 4 ε), 7,65 (mt, 1H: 1' H₆), 8,58 (d, J=9,5 Hz, 1H: NH 6).

### Exemple F

### Préparation de la 4ζ-tert-butyl-dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 5 g/l dans la soude 0,1N de 4-tert-butylphénylalanine(R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par palliers de 0 à 10 % de méthanol dans le dichlorométhane. Les fractions contenant le nouveau dérivé de pristinamycine I_{A} sont regroupées et évaporées. Le résidu sec est repris par 7 ml d'un mélange 60 % eau et 40 % acétonitrile et injecté en 2 fois sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 30 mg de 4ζ-tertbutyl-dés(4ζ-diméthylamino) pristinamycine I_{A}.

Spectre de R.M.N. ¹ H (400 MHz, CDCl₃, δ en ppm, réf. TMS): 0,21 (dd, J = 16 et 5,5 Hz, 1H, 5 β₂), 0,91 (t, J = 7,5 Hz, 3H: CH₃ 2 γ), 1,17 (mt, 1H: 3 β₂), de 1,20 à 1,40 (mt, 1H: 3 γ₂), 1,33 (s, 9H: CH₃ du tert-butyle), 1,35 (d, J = 7,5 Hz, 3H: CH₃ 1 γ), de 1,50 à 1,85 (mt, 3H: 3 γ₁ et CH₂ 2 β), 2,04 (mt, 1H, 3 β₁), 2,13 (mt, 1H, 5 δ₂), 2,30 (mt, 2H: 5 δ₁ et 5 β₁), 2,80 (dt, J = 13 et 4 Hz, 1H: 5 ε₂), 3,00 (dd, J = 12 et 4 Hz, 1H: 4 β₂), 3,29 (s, 3H: NCH₃ 4), 3,31 et 3,59 (2 mts, 1H chacun: CH₂ 3 δ), 3,40 (t, J = 12 Hz, 1H: 4 β₁), 4,57 (t, J = 7,5 Hz, 1H, 3 α), 4,74 (dd large, J = 13 et 7 Hz, 1H: 5 ε₁), 4,85 (mt, 1H: 2α), 4,90 (d large, J = 10 Hz, 1H: 1α), 5,21 (d large, J = 5,5 Hz, 1H: 5 α), 5,25 (dd, J = 12 et 4 Hz, 1H: 4 α), 5,87(d, J = 9 Hz, 1H: 6 α), 5,92 (q large, J = 7,5 Hz, 1H: 1β), 6,56 (d, J = 9,5 Hz, 1H: NH 2), de 7,10 à 7,30 (mt, 5H: H Aromatiques 6), 7,28 (d, J = 7,5 Hz, 2H: 4δ), 7,38 (d, J = 7,5 Hz, 2H: 4ε), 7,49 (d large, J = 8,5 Hz, 1H: 1' H₄), 7,53 (dd, J = 8,5 et 4 Hz, 1H: 1' H₅), 7,86 (d, J = 4 Hz, 1H: 1' H₆), 8,45 (d, J = 10 Hz, 1H: NH 1), 8,74 (d, J = 9 Hz, 1H: NH 6), 11,65 (s, 1H: OH).

Le chlorhydrate de 4-tert-butylphénylalanine (R,S) peut être préparé de la manière suivante :

Dans un tricol surmonté d'un réfrigérant sont additionnés 25 g de 4-(tert butyl) benzyl acétamidomalonate de diéthyle et 250 ml d'acide chlorhydrique à 37 %. Le mélange est agité et chauffé au reflux jusqu'à ce qu'il n'y ait plus de dégagement gazeux. Après refroidissement du mélange réactionnel le précipité obtenu est filtré puis recristallisé dans l'acétonitrile pour donner 25,6 g de chlorhydrate de 4-tert-butylphénylalanine (R,S) sous forme d'un solide blanc fondant à 234°C.

Le 4-(tert butyl) benzyl acétamidomalonate de diéthyle peut être préparé de la manière suivante :

Dans un tricol surmonté d'un réfrigérant sont additionnés sous atmosphère d'azote, 25 g de bromure de 4-tert-butyl benzyle, 50 ml de toluène anhydre et 3,1 g d'hydrure de sodium en suspension dans l'huile à 80 % puis 21,8 g d'acétamido malonate de diéthyle. Le mélange est chauffé à 110°C pendant 17 heures. Après refroidissement on ajoute lentement à l'aide d'une ampoule de coulée, 15 ml d'éthanol absolu puis 15 ml d'éthanol à 50 % puis 50 ml d'eau. La phase organique est décantée et la phase aqueuse lavée par 3 fois 50 ml d'éther diéthylique. Les phases organiques sont réunies, lavées à l'eau puis séchées sur sulfate de sodium. Après filtration et concentration sous pression réduite, le produit est cristallisé dans l'éther de pétrole pour donner 25 g de 4-(tert-butyl) benzyl acétamidomalonate de diéthyle sous forme d'un solide blanc fondant à 80°C.

Le mutant SP92::pVRC508 a été cultivé en milieu de production liquide. La fermentation a été réalisée comme suit : 0,5 ml d'une suspension de spores de la souche précitée est ajouté en conditions stériles à 40 ml de milieu inoculum dans un erlen chicané de 300 ml. Le milieu inoculum est constitué par 10 g/l de Corn Steep, 15 g/l de saccharose, 10 g/l de (NH₄)₂SO₄, 1 g/l de K₂HPO₄, 3 g/l de NaCl, 0,2 g/l de MgSO₄-7H₂O et 1,25 g/l de CaCO₃. Le pH est ajusté à 6,9 par de la soude avant l'introduction du carbonate de calcium. Les erlens sont agités pendant 44 heures à 27°C sur un agitateur rotatif à la vitesse de 325 rpm. 2,5 ml de la culture précédente âgée de 44 heures sont ajoutés stérilement à 30 ml de milieu de production dans un erlen de 300 ml. Le milieu de production est constitué par 25 g/l de farine de soja, 7,5 g/l d'amidon, 22,5 g/l de glucose, 3,5 g/l de levure fourragère, 0,5 g/l de sulfate de zinc et 6 g/l de carbonate de calcium. Le pH est ajusté à 6,0 par de l'acide chlorhydrique avant l'introduction du carbonate de calcium. Les erlens sont agités à 27°C sur un agitateur rotatif à la vitesse de 325. rpm. Au bout de 16 heures, 1 ml d'une solution d'un des précurseurs listés dans le tableau 3 (généralement 5 ou 10 g/l) est ajouté à la culture. Celle-ci est arrêtée 8 ou 24 heures plus tard. Aussitôt le moût est volumé et on lui ajoute 2 volumes de phase mobile composée de 34 % d'acétonitrile et 66 % d'une solution de 0,1 M de KH₂PO₄ (ajusté à pH 2,9 par H₃PO₄ concentré) permettant l'extraction des pristinamycines. Après agitation, le tout est centrifugé et les pristinamycines contenues dans le surnageant sont extraites et purifiées. Elles sont également dosées par CLHP en injectant 150 µl du surnageant de centrifugation sur une colonne Nucléosil 5-C8 de 4,6 x 150 mm éluée par un mélange de 40 % d'acétonitrile et de 60 % de tampon phosphate 0,1 M pH 2,9.

### Exemple G

### Préparation de la 4ζ-isopropyl-dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 10 g/l dans la soude 0,1N de 4-isopropylphénylalanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 61 mg de résidu sec. Celui-ci est repris par 9 ml d'un mélange 60 % eau et 40 % acétonitrile et injecté en 3 fois sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 51 mg de 4ζ-isopropyl-dés(4ζ-diméthylamino) pristinamycine I_{A}.

Spectre de R.M.N. ¹ H (250 MHz, CDCl₃, δ en ppm, réf. TMS): 0,31 (dd, J = 16 et 5,5 Hz, 1H, 5 β₂), 0,91 (t, J = 7,5 Hz, 3H: CH₃ 2 γ), de 1,00 à 1,45 (mt, 2H: 3 β₂ et 3 γ₂), 1,25 (d, J = 7,5 Hz, 6H: CH₃ de l'isopropyle), 1,35 (d, J = 7,5 Hz, 3H: CH₃ 1 γ), de 1,50 à 1,85 (mt, 3H: 3 γ₁ et CH₂ 2 β), de 1,95 à 2,20 (mt, 2H, 3 β₁ et 5 δ₂), 2,30 (mt, 2H: 5 δ₁ et 5 β₁), 2,80 (dt, J = 13 et 4 Hz, 1H: 5 ε₂), 2,88 (mt, 1H: CH de l' isopropyle), 2,98 (dd, J = 12 et 4 Hz, 1H: 4 β₂), 3,30 (s, 3H: NCH₃ 4), 3,32 et 3,55 (2 mts, 1H chacun: CH₂ 3 δ) , 3,38 (t, J = 12 Hz, 1H: 4 β₁), 4,55 (t, J = 7,5 Hz, 1H, 3 α), 4,72 (dd large, J = 13 et 7 Hz, 1H: 5 ε₁), 4,85 (mt, 1H: 2α), 4,88 (d large, J = 10 Hz, 1H: 1α), 5,21 (d large, J = 5,5 Hz, 1H: 5 α), 5,25 (dd, J = 12 et 4 Hz, 1H: 4 α), 5,87(d, J = 9 Hz, 1H: 6 α), 5,90 (q large, J = 7,5 Hz, 1H: 1β), 6,50 (d, J = 9,5 Hz, 1H: NH 2), de 7,05 à 7,35 (mt, 9H: H Aromatiques 6 - 4ε et 4δ), 7,50 (mt, 2H: 1' H₅ et 1' H₄), 7,86 (dd, J = 4 et 1,5 Hz, 1H: 1' H₆), 8,40 (d, J = 10 Hz, 1H: NH 1), 8,72 (d, J = 9 Hz, 1H: NH 6), 11,60 (s, 1H: OH).

La 4-isopropylphénylalanine (R,S) peut être préparée de la manière suivante :

On place dans un tricol 7 g de phosphore rouge et 8 g de 4-(isopropyl benzylidène) 2-méthyl 5-oxazolone dans 45 ml d'anhydride acétique puis on ajoute lentement sous agitation à l'aide d'une ampoule de coulée 35 ml d'acide iodhydrique à 57 %. En fin d'addition le mélange est chauffé 3 heures 30 minutes au reflux puis laissé 3 jours à température ambiante. Le mélange réactionnel est filtré, le solide obtenu rincé par 2 fois 10 ml d'acide acétique puis le filtrat concentré à sec sous pression réduite. Le résidu obtenu est repris par 100 ml d'eau distillée, concentré à sec sous pression réduite pour donné un solide qui est repris dans 50 ml d'eau distillée puis extrait par 3 fois 50 ml d'éther diéthylique après addition de 0,5 g de sulfite de sodium. L'éther est décanté et la phase aqueuse placée sous pression réduite pour éliminer les traces d'éther diéthylique. On ajoute à la phase aqueuse 2 g de noir animal, on chauffe à 40-50°C, on filtre sur Clarcel puis on rince avec un minimum d'eau. Le pH est ajusté à 5 par addition, à 4°C, d'ammoniaque à 32 %. Le précipité obtenu est filtré à froid, rincé par 2 fois 10 ml d'eau, 10 ml d'éthanol puis par 2 fois 10 ml d'éther pour donner après sèchage sous pression réduite à 20°C, 3,97 g de 4-isopropyl-phénylalanine (R,S) sous forme d'un solide blanc fondant à une température supérieure à 260°C.

La 4-(isopropyl benzylidène) 2-méthyl 5-oxazolone peut être préparée de la manière suivante :

On place dans un ballon muni d'un réfrigérant, 18,52 g de N-acétyl-glycine, 10,6 g d'acétate de sodium, 20 ml de 4-isopropyl-benzaldéhyde et 57 ml d'anhydride acétique. On agite 30 minutes puis le mélange est agité 1 heure à 110°C puis 15 heures à température ambiante. Le mélange réactionnel est versé sur 600 ml d'eau et 400 ml d'éther de pétrole préalablement chauffé à 50°C. La phase organique est décantée et la phase aqueuse lavée par 2 fois 150 ml d'éther de pétrole.

Les phases organiques sont rassemblées, séchées sur sulfate de magnésium, filtrées et concentrées sous pression réduite jusqu'à 100 ml et obtention d'un précipité. Celui-ci est filtré, lavé par 2 fois 50 ml de pentane pour donner 8,2 g de 4-(isopropyl benzylidène) 2-méthyl 5-oxazolone sous forme d'un solide jaune fondant à 77°C.

### Exemple H

### Préparation de la 4ζ-méthoxy-dés(4ζ-diméthylamino) pristinamycine I_{A}.

On réalise à l'échelle de 12 erlenmeyers une culture de la souche SP92::pVRC508 en milieu de production avec ajout à 16 heures de 1 ml d'une solution à 5 g/l dans la soude 0,1N de 4-méthoxyphénylalanine (R,S). Au terme de 40 heures de culture, les 0,35 litres de moût issus de 12 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volumes de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 14 mg de résidu sec. Celui-ci est repris par 3 ml d'un mélange 60 % eau et 40 % acétonitrile et injecté sur une colonne semi-préparative Nucléosil 7µ C8 10x250 mm (Macherey Nagel) éluée dans un mélange de 60 % de tampon phosphate 100 mM pH 2,9 et 40 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 12 mg de 4ζ-méthoxy-dés(4ζ-diméthylamino) pristinamycine I_{A}.

Spectre de R.M.N. ¹ H (400 MHz, CDCl₃, d en ppm, réf. TMS): 0,63 (dd, J = 16 et 5,5 Hz, 1H, 5 β₂), 0,96 (t, J = 7,5 Hz, 3H: CH₃ 2 γ), 1,17 (mt, 1H: 3 β₂), de1,30 à 1,45 (mt, 1H: 3 γ₂), 1,38 (d, J = 7,5 Hz, 3H: CH₃ 1 γ), de 1,55 à 1,85 (mt, 3H: 3 γ₁ et CH₂ 2 β), 2,05 (mt, 1H, 3 β₁), 2,20 (mt, 1H, 5 δ₂), 2,40 (d large, J = 16 Hz, 1H: 5 δ₁), 2,47 (d, J = 16 Hz, 1H: 5 β₁), 2,88 (dt, J = 13 et 4 Hz, 1H: 5 ε₂), 2,99 (dd, J = 12,5 et 5 Hz, 1H: 4 β₂), 3,30 (s, 3H: NCH₃ 4), 3,32 et 3,60 (2 mts, 1H chacun: CH₂ 3 δ), 3,40 (t, J = 12,5 Hz, 1H: 4 β₁), 3,80 (s, 3H: OCH₃), 4,60 (t, J = 7,5 Hz, 1H, 3 α), 4,80 (dd large, J = 13 et 8,5 Hz, 1H: 5 ε₁), 4,88 (mt, 1H: 2α), 4,92 (d large, J = 10 Hz, 1H: 1α) , 5,31 (dd, J = 12,5 et 5 Hz, 1H: 4 a), 5,34 (d large, J = 5,5 Hz, 1H: 5 α), 5,90(d, J = 9 Hz, 1H: 6 α), 5,93 (q large, J = 7,5 Hz, 1H: 1β), 6,54 (d, J = 9 Hz, 1H: NH 2), 6,87 (d, J = 8 Hz, 2H: 4ε), 7,16 (d, J = 8 Hz, 2H: 4δ), de 7,15 à 7,40 (mt, 5H: H Aromatiques 6), 7,50 (mt, 2H: 1' H₅ et 1' H₄), 7,80 (dd, J = 4 et 2,5 Hz, 1H: 1' H₆), 8,43 (d, J = 10 Hz, 1H: NH 1), 8,78 (d, J = 9 Hz, 1H: NH 6), 11,65 (s, 1H: OH).

### Exemple I

### Préparation de la 4ζ-méthyl-dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 5 g/l dans la soude 0,1N de 4-méthylphénylalanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 49 mg de résidu sec. Celui-ci est repris par 6 ml d'un mélange 60 % eau et 40 % acétonitrile et injecté en 2 fois sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 44 mg de 4ζ-méthyl-dés(4ζ-diméthylamino) pristinamycine I_{A}.

Spectre de R.M.N. ¹ H (400 MHz, CDCl₃, δ en ppm, réf. TMS): 0,52 (dd, J = 16 et 6 Hz, 1H, 5 β₂), 0,93 (t, J = 7,5 Hz, 3H: CH₃ 2 γ), 1,15 (mt, 1H: 3 β₂), de1,20 à 1,40 (mt, 1H: 3 γ₂), 1,35 (d, J = 7,5 Hz, 3H: CH₃ 1 γ), de 1,50 à 1,85 (mt, 3H: 3 γ₁ et CH₂ 2 β), 2,04 (mt, 1H, 3 β₁), 2,18 (mt, 1H, 5 δ₂), de 2,25 à 2,45 (mt, 2H: 5 δ₁ et 5 β₁), 2,36 (s, 3H: ArCH₃), 2,83 (dt, J = 13 et 4 Hz, 1H: 5 ε₂), 2,99 (dd, J = 13 et 4 Hz, 1H: 4 β₂) 3,28 (s, 3H: NCH₃ 4), 3,31 et 3,59 (2 mts, 1H chacun: CH₂ 3 δ), 3,40 (t, J = 13 Hz, 1H: 4 β₁), 4,59 (t, J = 7,5 Hz, 1H, 3 α), 4,74 (dd large, J = 13 et 7 Hz, 1H: 5 ε₁), 4,85 (mt, 1H: 2α), 4,89 (d large, J = 10 Hz, 1H: 1α), de 5,25 à 5,35 (mt, 2H: 5 α et 4 α), de 5,85 à 5,95 (mt, 2H: 6 α et 1β), 6,52 (d, J = 9,5 Hz, 1H: NH 2), 7,14 (AB limite, J = 9 Hz, 4H: 4δ et 4ε), de 7,15 à 7,35 (mt, 5H: H Aromatiques 6), 7,50 (mt, 2H: 1' H₄ et 1' H₅), 7,81 (dd, J = 4 et 2Hz, 1H: 1' H₆), 8,41 (d, J = 10 Hz, 1H: NH 1), 8,74 (d, J = 9 Hz, 1H: NH 6), 11,63 (s, 1H: OH).

### Exemple J

### Préparation de la 4ζ-méthylthio-dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 10 g/l dans la soude 0,1N de 4-méthylthiophénylalanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 65 mg de résidu sec. Celui-ci est repris par 6 ml d'un mélange 60 % eau et 40 % acétonitrile et injecté en 2 fois sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 45 mg de 4ζ-méthylthio-dés(4ζ-diméthylamino) pristinamycine I_{A}.

Spectre de R.M.N. ¹ H (400 MHz, CDCl₃, δ en ppm, réf. TMS): 0,68 (dd, J = 16 et 5,5 Hz, 1H, 5 β₂), 0,93 (t, J = 7,5 Hz, 3H: CH₃ 2 γ), 1,13 (mt, 1H: 3 β₂), de 1,25 à 1,40 (mt, 1H: 3 γ₂), 1,33 (d, J = 7,5 Hz, 3H: CH₃ 1 γ), de 1,55 à 1,85 (mt, 3H: 3 γ₁ et CH₂ 2 β), 2,02 (mt, 1H, 3 β₁), 2,18 (mt, 1H, 5 δ₂), 2,38 (d large, J = 16,5 Hz, 1H: 5 δ₁), 2,46 (s, 3H: SCH₃), 2,48 (d, J=16 Hz, 1H, 5 β₁), 2,85 (dt, J = 13,5 et 4 Hz, 1H: 5 ε₂), 3,00 (dd, J = 12 et 5 Hz, 1H: 4 β₂), 3,23 (s, 3H: NCH₃ 4), 3,37 (t, J = 12 Hz, 1H: 4 β₁), 3,37 et 3,58 (2 mts, 1H chacun: CH₂ 3 δ), 4,55 (t, J = 7,5 Hz, 1H, 3 α), 4,77 (dd large, J = 13,5 et 8 Hz, 1H: 5 ε₁), 4,86 (mt, 1H: 2α), 4,89 (dd, J = 10 et 1,5 Hz, 1H: 1α), 5,30 (d large, J = 5,5 Hz, 1H: 5 α), 5,32 (dd, J = 12 et 5 Hz, 1H: 4 α), 5,90 (d, J = 9,5 Hz, 1H: 6 α), 5,92 (dq, J = 7,5 et 1,5 Hz, 1H:1β), 6,55 (d, J = 9,5 Hz, 1H: NH 2), 7,13 (d, J = 8 Hz, 2H: 4δ), de 7,15 à 7,35 (mt, 5H: H Aromatiques 6), 7,19 (d, J = 8 Hz, 2H: 4ε), 7,45 (mt, 2H: 1' H₄ et 1' H₅), 7,76 (t, J = 5 Hz, 1H: 1' H₆), 8,42 (d, J = 10 Hz, 1H: NH 1), 8,76 (d, J = 9,5 Hz, 1H: NH 6), 11,65 (s, 1H: OH).

La 4-méthylthiophénylalanine (RS) peut être préparée selon la méthode décrite par R.L.Colescott, R.R.Herr, J.P. Dailey J. Am. Chem. Soc, 1957, 79, 4232-4235.

### Exemple K

### Préparation de la 4ε-méthylamino dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 10 g/l dans l'eau de 3-méthylaminophénylalanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 19 mg de résidu sec. Celui-ci est repris par 3 ml d'un mélange 60 % eau et 40 % acétonitrile et injecté sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 8 mg de 4ε-méthylamino-dés(4ζ-diméthylamino) pristinamycine I_{A}.

Spectre de R.M.N. ¹ H (400 MHz, CDCl₃, δ en ppm, réf. TMS): 0,93 (t, J = 7,5 Hz, 3H: CH₃ 2 γ), 1,00 (dd, J = 16 et 6 Hz, 1H, 5 β₂), 1,17 (mt, 1H: 3 β₂), de 1,25 à 1,40 (mt, 2H: 3 γ₂), 1,35 (d, J = 7,5 Hz, 3H: CH₃ 1 γ), de 1,55 à 1,80 (mt, 3H: 3 γ₁ et CH₂ 2 β), 2,03 (mt, 1H, 3 β₁), 2,23 (mt, 1H, 5 δ₂), 2,39 (d large, J = 16 Hz, 1H: 5 δ₁), 2,52 (d, J = 16 Hz, 1H: 5 β₁), 2,78 (s, 3H: ArNCH₃ 4), 2,85 (dt, J = 13 et 4 Hz, 1H: 5 ε₂), 2,99 (dd, J = 13 et 4,5 Hz, 1H: 4 β₂), 3,23 (s, 3H: NCH₃ 4), 3,25 (t, J = 13 Hz, 1H: 4 β₁), 3,38 et 3,58 (2 mts, 1H chacun: CH₂ 3 δ), 4,05 (mf, 1H: ArNH), 4,58 (dd, J = 6,5 et 7,5 Hz, 1H, 3 α), 4,76 (dd large, J = 13 et 8 Hz, 1H: 5 ε₁), 4,85 (mt, 1H: 2α), 4,87 (d large, J = 10 Hz, 1H: 1α), 5,35 (dd, J = 13 et 4,5 Hz, 1H: 4 α), 5,38 (d large, J = 6 Hz, 1H: 5 α), 5,90 (d, J=9,5 Hz, 1H: 6 α), 5,91 (mt, 1H: 1β), 6,36 (s large, 1H: H 2 de l'aromatique en 4), de 6,45 à 6,55 (mt, 2H: H 4 et H 6 de l'aromatique en 4), 6,53 (d, J = 10 Hz, 1H: NH 2), 7,12 (t, J = 8 Hz, 1H: H 5 de l'aromatique en 4), de 7,15 à 7,45 (mt, 5H: H Aromatiques 6), 7,35 (mt, 2H: 1' H₄ et 1' H₅), 7,75 (t, J = 3 Hz, 1H: 1' H₆), 8,40 (d, J = 10 Hz, 1H: NH 1), 8,78 (d, J = 9,5 Hz, 1H: NH 6), 11,60 (s, 1H: OH).

Le dichlorhydrate de 3-méthylaminophénylalanine (R,S) peut être préparé de la manière suivante :

En opérant comme à l'exemple F mais à partir de 1,17 g 3-méthylamino benzyl acétamidomalonate de diéthyle et 20 ml d'acide chlorhydrique 12 N, on obtient 1,03 g d'un solide jaune beige. Celui-ci est dissous dans 20 ml d'éthanol absolu et additionné de 0,4 g de noir animal. La solution est filtrée sur Clarcel, puis filtrée et concentrée sous pression réduite (50 kPa) La même opération est recommencée avec 1 g de noir animal et le solide obtenu trituré dans 20 ml d'éther. Aprés filtration et séchage sous pression réduite (2,7 kPa) à 50°C, on obtient 0,65 g de dichlorhydrate de 3-méthylaminophénylalanine (R,S) sous forme d'une poudre blanche fondant vers 135°C (décomposition).

Le 3-méthylamino benzyl acétamidomalonate de diéthyle peut être préparé de la manière suivante :

On place dans un tricol maintenu sous atmosphère d'azote 3,11 ml d'anhydride acétique. On ajoute ensuite en 3 minutes à 0°C, 1,51 ml d'acide formique puis on chauffe à 50°C pendant 2 heures. On laisse le mélange revenir à température ambiante en agitant pendant 3 heures 20 minutes et on ajoute 4 ml de THF anhydre sous azote et on refroidit à -20°C. On ajoute en 10 minutes, une solution de 4 g de 3-aminobenzyl acétamidomalonate de diéthyle dans un mélange de 15 ml de THF anhydre et de 15 ml de dichlorométhane anhydre. L'agitation est poursuivie 1 heure 10 minutes à -20°C puis à 20°C pendant 16 heures. Le mélange réactionnel est concentré à sec sous pression réduite (50 kPa) à 30°C, puis coévaporé avec 30 ml de toluène anhydre pour donner un solide blanc qui est dissous dans un mélange de 10 ml de THF anhydre et de 20 ml de dichloro 1,2 éthane anhydre puis placé dans un tricol sous azote.

Le mélange est refroidi à -5°C puis 1,55 ml de complexe borane-diméthylsulfure (solution 2M dans le THF) est ajouté en 10 minutes. On laisse le mélange revenir à température ambiante et la solution et chauffée 3 heures au reflux puis agitée 15 heures à température ambiante. Le mélange réactionnel est refroidi à 0°C puis on ajoute en 25 minutes, 10 ml de MeOH. On agite 45 minutes à 0°C puis 30 minutes à température ambiante. On refroidit à 0°C puis on fait barboter HCl gaz jusqu'à pH 2. On chauffe 1 heure à reflux puis le mélange est concentré à sec sous pression réduite à 30°C pour donner 5 g d'un produit qui est repris par 30 ml d'une solution aqueuse de NaHCO₃ et de 30 ml de CH₂Cl₂. La phase organique est décantée et la phase aqueuse lavée par 20 ml d'eau. Les phases organiques sont réunies, séchées sur sulfate de magnésium, filtrées puis concentrées à sec sous pression réduite (2,6 kPa) pour donner 3,43 g d'une huile jaune qui est purifiée par chromatographie flash (éluant AcOEt-cyclohexane 50/50). On obtient ainsi après séchage sous pression réduite (2,7 kPa) à 20°C, 1,18 g de 3-méthylamino benzyl acétamidomalonate de diéthyle sous forme d'un solide beige clair fondant à 122°C.

Le 3-amino benzyl acétamidomalonate de diéthyle peut être préparé comme décrit par T.S. Osdene, D.N.Ward, W.H. Chapman et H. Rakoff, J. Am. Chem. Soc., 81, 1959, 3100-3102.

### Exemple L

### Préparation de la 4ζ-trifluorométhyl dés(4ζ-diméthylamino) pristinamycine I_{A}.

On réalise à l'échelle de 60 erlenmeyers une culture de la souche SP92::pVRC508 en milieu de production avec ajout à 16 heures de 1 ml d'une solution à 5 g/l dans la soude 0,1N de 4-trifluorométhylphénylalanine (S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 3 ml d'un mélange 60 % eau et 40 % acétonitrile et injecté sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 5 mg de 4ζ-trifluorométhyl dés(4ζ-diméthylamino) pristinamycine I_{A}.

Spectre de R.M.N. ¹ H (400 MHz, CDCl₃, δ en ppm, réf. TMS): 0,86 (dd, J = 16 et 5,5 Hz, 1H, 5 β₂), 0,91 (t, J = 7,5 Hz, 3H: CH₃ 2 γ), 1,13 (mt, 1H: 3 β₂), 1,31 (d, J = 7,5 Hz, 3H: CH₃ 1 γ), 1,42 (mt, 1H: 3 γ₂), de 1,55 à 1,80 (mt, 3H: 3 γ₁ et CH₂ 2 β), 2,02 (mt, 1H, 3 β₁), 2,15 (mt, 1H, 5 δ₂), 2,40 (d large, J = 16,5 Hz, 1H: 5 δ₁), 2,55 (d, J=16 Hz, 1H, 5 β₁), 2,88 (dt, J = 14 et 4 Hz, 1H: 5 ε₂), 3,18 (s, 3H: NCH₃ 4), 3,20 et 3,31 (2 dd, respectivement J = 13 et 6 Hz et J = 13 et 10 Hz,, 1H chacun: 4 β₂ et 4 β₁), 3,42 et 3,60 (2 mts, 1H chacun: CH₂ 3 δ), 4,50 (t, J = 7,5 Hz, 1H, 3 α), 4,73 (dd large, J = 14 et 7,5 Hz, 1H: 5 ε₁), 4,83 (mt, 1H: 2α), 4,91 (d large, J = 10 Hz, 1H: 1α), 5,40 (d large, J = 5,5 Hz, 1H: 5 α), 5,55 (dd, J = 10 et 6 Hz, 1H: 4 α), 5,87 (d, J = 9,5 Hz, 1H: 6 α), 5,90 (q large, J = 7,5 Hz, 1H:1β), 6,68 (d, J = 9,5 Hz, 1H: NH 2), de 7,15 à 7,40 (mt, 9 H: 4δ - H Aromatiques 6 - 1' H₅ et 1' H₄), 7,52 (d, J = 8 Hz, 2H: 4ε), 7,68 (d, J = 4 et 1,5 Hz, 1H: 1' H₆), 8,43 (d, J = 10 Hz, 1H: NH 1), 8,76 (d, J = 9,5 Hz, 1H: NH 6), 11,70 (s, 1H: OH).

### Exemple M

### Préparation de la 4ε-méthoxy dés(4ζ-diméthylamino) pristinamycine IA.

On réalise à l'échelle de 60 erlenmeyers une culture de la souche SP92::pVRC508 en milieu de production avec ajout à 16 heures de 1 ml d'une solution à 5 g/l dans la soude 0,1N de 3-methoxyphénylalanine (S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine IA sont regroupées et évaporées. On obtient 41 mg de résidu sec. Celui-ci est repris par 6 ml d'un mélange 60 % eau et 40 % acétonitrile et injecté en 2 fois sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 28 mg de 4ε-méthoxy dés(4ζ-diméthylamino) pristinamycine I A.

Spectre de R.M.N. ¹ H (400 MHz, CDCl₃, δ en ppm, réf. TMS): 0,52 (dd, J = 16 et 5,5 Hz, 1H, 5 β₂), 0,90 (t, J = 7,5 Hz, 3H: CH₃ 2 γ), de 1,10 à 1,34 (mt, 2H: 3 β₂ et 3 γ₂), 1,34 (d, J = 7,5 Hz, 3H: CH₃ 1γ), de 1,50 à 1,80 (mt, 3H: 3 γ₁ et CH₂ 2 β), 2,04 (mt, 1H, 3 · β₁), 2,20 (mt, 1H, 5 δ₂), 2,35 (d large, J = 16 Hz, 1H: 5 δ₁), 2,38 (d, J = 16 Hz, 1H: 5 β₁), 2,83 (dt, J = 13 et 4 Hz, 1H: 5 ε₂), 2,97 (dd, J = 12 et 4 Hz, 1H: 4 β₂), 3,28 (s, 3H: NCH₃ 4), 3,28 et 3,56 (2 mts, 1H chacun: CH₂ 3 δ), 3,40 (t, J = 12 Hz, 1H: 4 β₁), 3,80 (s, 3H: OCH₃), 4,58 (t, J = 7,5 Hz, 1H, 3 α), 4,76 (dd large, J = 13 et 8 Hz, 1H: 5 ε₁), 4,85 (mt, 1H: 2α), 4,90 (d large, J = 10 Hz, 1H: 1α), 5,27 (dd, J = 12 et 4 Hz, 1H: 4 α), 5,30 (d large, J = 5,5 Hz, 1H: 5 α), 5,89 (d, J = 9,5Hz, 1H: 6 α), 5,91 (q large, J = 7,5 Hz, 1H: 1β), 6,51 (d, J = 10 Hz, 1H: NH 2), de 6,80 à 6,90 (mt, 3H: H 2 - H 4 et H 6 de l'aromatique en 4), de 7,15 à 7,40 (mt, 6H: H 5 de l'aromatique en 4 et H Aromatiques 6), 7,45 (d large, J = 9 Hz, 1H: 1' H₄), 7,50 (dd, J = 9 et 4 Hz, 1H:1' H₅), 7,80 (d large, J = 4 Hz, 1H: 1' H₆), 8,40 (d, J = 10 Hz, 1H: NH 1), 8,73 (d, J = 9,5 Hz, 1H: NH 6), 11,62 (s, 1H: OH).

### Exemple N

### Préparation de 4ε-fluoro 4ζ-méthyl dés(4ζ-diméthylamino) pristinamycine IA.

On réalise à l'échelle de 60 erlenmeyers une culture de la souche SP92::pVRC508 en milieu de production avec ajout à 16 heures de 1 ml d'une solution à 10 g/l dans la soude 0,1N de 3-fluoro 4-méthylphénylalanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par palliers de 0 à 10 % de méthanol dans le dichlorométhane. Les fractions contenant le nouveau dérivé de pristinamycine IA sont regroupées et évaporées. On obtient 15 mg de résidu sec. Celui-ci est repris par 3 ml d'un mélange 60 % eau et 40 % acétonitrile et injecté sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 9 mg de 4ε-fluoro 4ζ-méthyl-dés(4ζ-diméthylamino) pristinamycine I A.

Spectre de R.M.N. ¹ H (400 MHz, CDCl₃, δ en ppm, réf. TMS): 0,60 (dd, J = 16 et 5,5 Hz, 1H, 5 β₂), 0,91 (t, J = 7,5 Hz, 3H: CH₃ 2 γ), 1,12 (mt, 1H: 3 β₂), de 1,25 à 1,35 (mt, 1H: 3 γ₂), 1,33 (d, J = 7,5 Hz, 3H: CH₃ 1 γ), de 1,50 à 1,85 (mt, 3H: 3 γ₁ et CH₂ 2 β), 2,02 (mt, 1H, 3 β₁), 2,13 (mt, 1H, 5 δ₂), 2,27 (s, 3H: ArCH₃), 2,36 (d large, J = 16 Hz, 1H: 5 δ₁), 2,45 (d, J = 16 Hz, 1H: 5 β₁), 2,85 (dt, J = 13 et 4,5 Hz, 1H: 5 ε₂), 2,97 (dd, J = 12,5 et 4,5 Hz, 1H: 4 β₂), 3,23 (s, 3H: NCH₃ 4), 3,30 et 3,56 (2 mts, 1H chacun: CH₂ 3 δ), 3,37 (t, J = 12,5 Hz, 1H: 4 β₁), 4,55 (t, J = 7,5 Hz, 1H, 3 α), 4,75 (dd large, J = 13 et 8 Hz, 1H: 5 ε₁), 4,83 (mt, 1H: 2α), 4,89 (d large, J = 10 Hz, 1H: 1α), 5,29 (dd, J = 12,5 et 4,5 Hz, 1H: 4 α), 5,32 (d large, J = 5,5 Hz, 1H: 5 α), 5,89 (d, J = 9,5 Hz, 1H: 6 α), 5,92 (mt, 1H: 1β), 6,49 (d, J = 10 Hz, 1H: NH 2), 6,90 (mt, 2H: H 2 et H 6 de l'aromatique en 4), 7,11 (t, J = 8 Hz, 1H: H 5 de l'aromatique en 4), de 7,10 à 7,30 (mt, 5H: H Aromatiques 6), 7,43 (dd, J = 8,5 et 1 Hz, 1H: 1' H₄), 7,49 (dd, J = 8,5 et 4,5 Hz, 1H: 1' H₅), 7,75 (dd, J = 4,5 et 1Hz, 1H: 1' H₆), 8,48 (d, J = 10 Hz, 1H: NH 1), 8,70 (d, J = 9,5 Hz, 1H: NH 6), 11,60 (s, 1H: OH).

Le chlorhydrate de 3-fluoro 4-méthylphénylalanine (R,S) peut être préparé de la manière suivante :

A 1,6 g de N-acétyl (3-fluoro-4 méthyl) phénylalaninate de méthyle on ajoute de l'acide chlorhydrique 12 N et le mélange est chauffé au reflux sous agitation pendant 8 heures. Après une nuit à température ambiante, le mélange réactionnel est concentré à sec sous pression réduite (50 kPa), repris par un mélange 50/50 en volumes de toluène et d'éthanol, puis à nouveau concentré à sec. Après séchage sous pression réduite (2,6 kPa), on obtient 0,6 g de chlorhydrate de 3-fluoro 4-méthyl phénylalanine (R,S) sous forme de cristaux blancs fondant à une température supérieure à 260°C.

Le N-acétyl (3-fluoro-4 méthyl) phénylalaninate de méthyle (R,S) peut être préparé de la manière suivante :

A 1,9 g de (4-méthyl 3-fluoro) 2-acétamido cinnamate de méthyle placé sous atmosphère d'azote dans un autoclave, on ajoute 0,2 g de palladium sur charbon à 10 % dans 230 ml d'éthanol absolu. Le mélange est placé sous une pression de 5,5 bars d'hydrogène et chauffé 15 heures à 50°C sous agitation. Après stabilisation de la température à 26°C et remise à pression atmosphérique, le mélange réactionnel est filtré sur Clarcel® , rincé à l'éthanol puis concentré à sec sous sous pression réduite (2,6 kPa). On obtient 1,6 g de N-acétyl (3-fluoro-4 méthyl) phénylalaninate de méthyle sous forme d'une huile incolore (Silice Merck 5719, Rf= 0,46; éluant CH₂Cl₂/AcOEt 50/50).

Le (3-fluoro 4-méthyl) 2-acétamido cinnamate de méthyle peut être préparé de la manière suivante :

Dans un tricol placé sous azote on ajoute 3,6 g de 2-acétamido acrylate de méthyle, 0,12 g d'acétate de palladium, 5,2 g de chlorure de tétrabutyl ammonium et 3,8 g d'hydrogénocarbonate de sodium puis on additionne à ce mélange 4 g de 2-fluoro 4-bromo toluène en solution dans 120 ml de DMF anhydre. Le mélange est chauffé 16 heures 30 minutes à 82°C puis après refroidissement versé sur 1000 ml d'eau distillée. Le mélange est repris par 250 ml de CH₂Cl₂, la phase organique est décantée et la phase aqueuse lavée par 2 fois 250 ml de CH₂Cl₂. Les phases organiques sont rassemblées, séchées sur sulfate de sodium, filtrées et concentrées sous pression réduite (50 kPa) à 70°C pour donner une huile brune qui est purifiée par flash-chromatographie (éluant AcOEt/cyclohexane puis AcOEt pur). On obtient 2,6 g de (3-fluoro 4-méthyl) 2-acétamido cinnamate de méthyle sous forme d'un solide blanc fondant à 163°C.

### Exemple O

### Préparation de la 4ε-éthoxy-dés(4ζ-diméthylamino) pristinamycine I_{A}.

On réalise à l'échelle de 60 erlenmeyers comme décrit dans l'exemple F une culture de la souche SP92::pVRC508 en milieu de production avec ajout à 16 heures de 1 ml d'une solution à 20 g/l dans la soude 0,2N de chlorhydrate de 3-O-éthyltyrosine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 19 mg de résidu sec. Celui-ci est repris par 3 ml d'un mélange 60 % eau et 40 % acétonitrile et injecté sur une colonne semi-préparative Nucléosil 7µ C8 10x250 mm (Macherey Nagel) éluée dans un mélange de 60 % de tampon phosphate 100 mM pH 2,9 et 40 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 15,8 mg de 4ε-O-éthoxy-dés(4ζ-diméthylamino) pristinamycine I_{A}.

Spectre de R.M.N. ¹ H (400 MHz, CDCl₃, δ en ppm, ref. TMS)

0,55 (dd, J = 16 et 5,5 Hz, 1H : 1H du CH₂ en 5 β) ; 0,90 (t, J = 7,5 Hz, 3H : CH₃ en 2 γ) ; 1,12 (mt, 1H : 1H du CH₂ en 3 β) ; 1,20 (mt, 1H : 1H du CH₂ en 3 γ) ; 1,31 (d, J = 6,5 Hz, 3H : CH₃ en 1 γ) ; 1,49 (t, J = 7 Hz, 3H : CH₃ de l'éthyle) ; 1,54 (mt, 1H : l'autre H du CH₂ en 3 γ) ; 1,63 et 1,73 (2 mts, 1H chacun : CH₂ en 2 β) ; 2,02 (mt, 1H : l'autre H du CH₂ en 3 β) ; 2,22 et 2,33 (respectivement mt et d large, J = 16,5 Hz, 1H chacun : CH₂ en 5 δ) ; 2,46 (d, J = 16 Hz, 1H : l'autre H du CH₂ en 5 β) ; 2,83 (dt, J = 13 et 4 Hz, 1H : 1H du CH₂ en 5 ε) ; 2,95 (dd, J = 12 et 4 Hz, 1H : 1H du CH₂ en 4 β) ; 3,22 (mt, 1H : 1H du CH₂ en 3 δ) ; 3,27 (s, 3H : NCH₃) ; 3,39 (t, J = 12 Hz, 1H : l'autre H du CH₂ en 4 β) ; 3,53 (mt, 1H : l'autre H du CH₂ en 3 δ) ; 3,93 et 4,03 (2 mts, 1H chacun : OCH₂ de l'éthyle) ; 4,56 (dd, J = 7 et 5,5 Hz, 1H : 3 α) ; 4,75 (dd large, J = 13 et 8 Hz, 1H : l'autre H du CH₂ en 5 ε) ; 4,82 (mt, 1H : 2 α) ; 4,88 (dd, J = 10 et 1 Hz, 1H : 1 α) ; 5,23 (dd, J = 12 et 4 Hz, 1H : 4 α) ; 5,23 (d large, J = 5,5 Hz, 1H : 5 α) ; 5,87 (d, J = 9,5 Hz, 1H : 6 α) ; 5,92 (mt, 1H : 1 β) ; 6,47 (d, J = 10 Hz, 1H : NH en 2) ; 6,80 (mt, 3H : H Aromatiques en ortho et en para de l'éthoxy) ; de 7,10 à 7,35 (mt, 6H : H Aromatiques en 6 et H Aromatiques en méta de l'éthoxy) ; 7,43 (dd, J = 8 et 1 Hz, 1H : 1' H₄) ; 7,50 (dd, J = 8 et 4 Hz, 1H : 1' H₅) ; 7,77 (dd, J = 4 et 1 Hz, 1H : 1' H₆) ; 8,38 (d, J = 10 Hz, 1H : NH en 1); 8,70 (d, J = 9,5 Hz, 1H : NH en 6) ; 11,60 (s, 1H : OH).

Le chlorhydrate de 3-O-éthyltyrosine (R,S) peut être préparé de la manière suivante :

On place dans un ballon 1 g de N-tert.butoxycarbonyl 3-éthoxy phénylalanine (R,S) en solution dans 3,6 ml de dioxanne chlorhydrique puis le mélange est agité 5 heures à température ambiante. Le précipité formé est filtré, rincé par du dioxanne puis à l'éther puis séché sous pression réduite (2,7 kPa) à 40°C pour donner 0,65 g de chlorhydrate de 3-O-éthyltyrosine (R,S) sous forme d'un solide blanc fondant à 200°C.

Le N-tert.butoxycarbonyl 3-éthoxy phénylalanine (R,S) peut être préparé de la manière suivante :

On place dans un ballon 1,33 g de N-tert.butoxycarbonyl 3-éthoxy phénylalaninate d'éthyle (R,S) en solution dans 8 ml de méthanol puis on ajoute 8 ml de soude 1N. Après 18 heures d'agitation à température ambiante, le mélange est évaporé sous pression réduite, puis acidifié par 8,56 ml d'acide chlorhydrique 1N. Le produit est extrait par 2 fois 10 ml d'acétate d'éthyle, les phases organiques sont réunies, lavées par 2 fois 10 ml d'eau, séchées filtrées puis concentrées à sec sous pression réduite pour donner 1 g de N-tert.butoxy carbonyl 3-éthoxy phénylalanine (R,S) sous forme d'une huile jaune (Silice Merck 5719, Rf = 0,7, éluant : toluène 80 / MeOH 10 / diéthylamine 10).

Le N-tert.butoxycarbonyl 3-éthoxy phénylalaninate d'éthyle (R,S) peut être préparé de la manière suivante :

On place dans un tricol sous atmosphère d'azote, 1,5 g de N-tert.butoxycarbonyl 3-tyrosine (R,S) en solution dans 7,5 ml de diméthylformamide sec puis on ajoute, 0,508 g d'hydrure de sodium à 50 % dans l'huile. Après 2 heures d'agitation à température ambiante on ajoute 0,86 ml de iodoéthane puis le mélange est agité 4 heures à température ambiante. Le milieu est filtré, le solide résultant lavé par 3 fois 10 ml d'eau puis 2 fois 10 ml d'éther de pétrole pour donner après séchage sous pression réduite (2,7 kPa) à 30°C, 1,33 g de N-tert.butoxycarbonyl 3-éthoxy phénylalaninate d'éthyle (R,S) sous forme d'un solide blanc.

La N-tert.butoxycarbonyl 3-tyrosine (R,S) peut être préparée de la manière suivante :

On place sous agitation dans un tricol, 18 g de 3-tyrosine (R,S) en solution dans 180 ml de dioxanne puis on ajoute 99 ml de soude 1N puis 26 g de di-tert.butyl dicarbonate en solution dans 160 ml de dioxanne. Après 36 heures d'agitation le milieu est concentré sous pression réduite à 30°C, repris avec 100 ml d'eau distillée, acidifié avec de l'acide chlorhydrique 1N jusqu'à pH 5 puis extrait par 2 fois 200 ml d'acétate d'éthyle. La phase organique est séchée sur sulfate de magnésium, filtrée puis concentrée à sec sous pression réduite à 30°C, pour donner 30 g de N-tert.butoxy carbonyl 3-tyrosine (R,S) sous forme d'un solide blanc (Silice Merck 5719, Rf = 0,25, éluant : toluène 80, MeOH 10, diéthylamine 10).

### Exemple P

### Préparation de la 4ζ-éthoxy-dés(4ζ-diméthylamino) pristinamycine I_{A}

On réalise à l'échelle de 90 erlenmeyers comme décrit dans l'exemple F une culture de la souche SP92::pVRC508 en milieu de production avec ajout à 16 heures de 1 ml d'une solution à 20 g/l dans l'acide chlorhydrique 0,1N de chlorhydrate de 4-O-éthyltyrosine (S). Au terme de 40 heures de culture, les 2,7 litres de moût issus de 90 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant la 4ζ-éthoxy-dés(4ζ-diméthylamino) pristinamycine I_{A} sont regroupées et évaporées. Le résidu sec est repris par 7 ml d'un mélange 60 % eau et 40 % acétonitrile et injecté sur une colonne semi-préparative Nucléosil 7µ C8 10x250 mm (Macherey Nagel) éluée dans un mélange de 52 % de tampon phosphate 100 mM pH 2,9 et 48 % d'acétonitrile. Les fractions contenant la 4ζ-éthoxy-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 29 mg de 4ζ-éthoxy-dés(4ζ-diméthylamino) pristinamycine I_{A}.

Spectre de R.M.N. ¹ H (400 MHz, CDCl₃, δ en ppm, ref. TMS)

0,64 (dd, J = 16 et 5,5 Hz, 1H : 1H du CH₂ en 5 β) ; 0,90 (t, J = 7,5 Hz, 3H : CH₃ en 2 γ) ; 1, 12 (mt, 1H : 1H du CH₂ en 3 β) ; 1,25 (mt, 1H : 1H du CH₂ en 3 γ) ; 1,33 (d, J = 7 Hz, 3H : CH₃ en 1 γ) ; 1,42 (t, J = 7 Hz, 3H : CH₃ de l'éthyle) ; 1,57 (mt, 1H : l'autre H du CH₂ en 3 γ) ; 1,63 et 1,74 (2 mts, 1H chacun : CH₂ en 2 β) ; 2,02 (mt, 1H : l'autre H du CH₂ en 3 β) ; 2,16 et 2,35 (respectivement mt et d large, J = 16,5 Hz, 1H chacun : CH₂ en 5 δ) ; 2,43 (d, J = 16 Hz, 1H : l'autre H du CH₂ en 5 β) ; 2,83 (dt, J = 13 et 4 Hz, 1H : 1H du CH₂ en 5 ε) ; 2,93 (dd, J = 12 et 4 Hz, 1H : 1H du CH₂ en 4 β) ; de 3,15 à 3,30 (mt, 1H : 1H du CH₂ en 3 δ) ; 3,24 (s, 3H : NCH₃) ; 3,35 (t, J = 12 Hz, 1H : l'autre H du CH₂ en 4 β) ; 3,55 (mt, 1H : l'autre H du CH₂ en 3 δ) ; 3,95 (AB limite, 2H : OCH₂ de l'éthyle) ; 4,56 (dd, J = 7,5 et 6 Hz, 1H : 3 α) ; 4,75 (dd large, J = 13 et 8 Hz, 1H : l'autre H du CH₂ en 5 ε) ; 4,84 (mt, 1H : 2 α) ; 4,87 (dd, J = 10 et 1 Hz, 1H : 1 α) ; 5,26 (dd, J = 12 et 4 Hz, 1H : 4 α) ; 5,32 (d large, J = 5,5 Hz, 1H : 5 α) ; 5,88 (d, J = 10 Hz, 1H : 6 α) ; 5,92 (mt, 1H : 1 β) ; 6,48 (d, J = 10 Hz, 1H : NH en 2) ; 6,83 (d, J = 8 Hz, 2H : H Aromatiques en 4 ε) ; 7,10 (d, J = 8 Hz, 2H : H Aromatiques en 4 δ) ; de 7,10 à 7,35 (mt, 5H : H Aromatiques en 6) ; 7,44 (dd, J = 8,5 et 1,5 Hz, 1H : 1' H₄) ; 7,57 (dd, J = 8,5 et 4,5 Hz, 1H : 1' H₅) ; 7,77 (dd, J = 4,5 et 1,5 Hz, 1H : 1' H₆) ; 8,38 (d, J = 10 Hz, 1H : NH en 1) ; 8,75 (d, J = 10 Hz, 1H : NH en 6) ; 11,60 (s, 1H : OH).

Le chlorhydrate de 4-O-éthyltyrosine (S) peut être synthétisé comme décrit par Y. Sasaki et coll, Chem. Pharm. Bull., 30, 4435 (1982).

### Exemple O

### Préparation de la 4ζ-allyloxy-dés(4ζ-diméthylamino) pristinamycine I_{A}

On réalise à l'échelle de 90 erlenmeyers comme décrit dans l'exemple F une culture de la souche SP92::pVRC508 en milieu de production avec ajout à 16 heures de 1 ml d'une solution à 20 g/l dans l'acide chlorhydrique 0,1N de chlorhydrate de 4-O-allyltyrosine (S). Au terme de 40 heures de culture, les 2,7 litres de moût issus de 90 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant la 4ζ-allyloxy-dés(4ζ-diméthylamino) pristinamycine I_{A} sont regroupées et évaporées. Le résidu sec est repris par 7 ml d'un mélange 60 % eau et 40 % acétonitrile et injecté sur une colonne semi-préparative Nucléosil 7µ C8 10x250 mm (Macherey Nagel) éluée dans un mélange de 52 % de tampon phosphate 100 mM pH 2,9 et 48 % d'acétonitrile. Les fractions contenant la 4ζ-allyloxy-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 29 mg de 4ζ-allyloxy-dés(4ζ-diméthylamino) pristinamycine I_{A}.

Spectre de R.M.N. ¹ H (400 MHz, CDCl₃, δ en ppm, ref. TMS)

0,63 (dd, J = 16 et 6 Hz, 1H : 1H du CH₂ en 5 β) ; 0,91 (t, J = 7,5 Hz, 3H : CH₃ en 2 γ) ; 1,13 (mt, 1H : 1H du CH₂ en 3 β) ; 1,29 (mt, 1H : 1H du CH₂ en 3 γ) ; 1,33 (d, J = 6,5 Hz, 3H : CH₃ en 1 γ) ; 1,57 (mt, 1H : l'autre H du CH₂ en 3 γ) ; 1,65 et 1,74 (2 mts, 1H chacun : CH₂, en 2 β) ; 2,02 (mt, 1H : l'autre H du CH₂ en 3 β) ; 2,14 et 2,34 (respectivement mt et d large, J = 16,5 Hz, 1H chacun : CH₂ en 5 δ) ; 2,43 (d, J = 16 Hz, 1H : l'autre H du CH₂ en 5 β) ; 2,85 (dt, J = 13 et 4 Hz, 1H : 1H du CH₂ en 5 ε) ; 2,95 (dd, J = 12 et 4 Hz, 1H : 1H du CH₂ en 4 β) ; 3,25 (s, 3H : NCH₃) ; 3,33 (mt, 1H : 1H du CH₂ en 3 δ) ; 3,36 (t, J = 12 Hz, 1H : l'autre H du CH₂ en 4 β) ; 3,56 (mt, 1H : l'autre H du CH₂ en 3 δ) ; 4,51 (AB limite, 2H : OCH₂ de l'allyle) ; 4,56 (t, J = 7,5 Hz, 1H : 3 α) ; 4,75 (dd large, J = 13 et 8 Hz, 1H : l'autre H du CH₂ en 5 ε) ; 4,84 (mt, 1H : 2 α) ; 4,88 (dd, J = 10 et 1 Hz, 1H : 1 α) ; 5,27 (dd, J = 12 et 4 Hz, 1H : 4 α) ; 5,32 (d large, J = 6 Hz, 1H : 5 α) ; 5,30 et 5,40 (respectivement mt et dd, J = 17 et 1,5 Hz, 1H chacun : =CH₂ de l'allyle) ; 5,89 (d, J = 9,5 Hz, 1H : 6 α) ; 5,91 (mt, 1H : 1 β) ; 6,02 (mt, 1H : CH= de l'allyle) ; 6,50 (d, J = 10 Hz, 1H : NH en 2) ; 6,85 (d, J = 8 Hz, 2H : H Aromatiques en 4 ε) ; 7,12 (d, J = 8 Hz, 2H : H Aromatiques en 4 δ) ; de 7,10 à 7,35 (mt, 5H : H Aromatiques en 6) ; 7,45 (dd, J = 8,5 et 1,5 Hz, 1H : 1' H₄) ; 7,57 (dd, J = 8,5 et 4 Hz, 1H : 1' H₅) ; 7,77 (dd, J = 4 et 1,5 Hz, 1H : 1' H₆) ; 8,41 (d, J = 10 Hz, 1H : NH en 1) ; 8,74 (d, J = 9,5 Hz, 1H : NH en 6) ; 11,63 (s, 1H : OH).

Le chlorhydrate de 4-O-allyltyrosine (S) peut être synthétisé comme décrit par A. Loffet, H. Zang, Int. J. Pept. Protein Res. ,42, 346 (1993).

### Exemple R

### Préparation de la 4ζ-éthylamino-dés(4ζ-diméthylamino) pristinamycine I_{A}

On réalise à l'échelle de 50 erlenmeyers comme décrit dans l'exemple F une culture de la souche SP92::pVRC508 en milieu de production avec ajout à 16 heures de 1 ml d'une solution à 20 g/l dans la soude 0,1N de dichlorhydrate de 4-éthylaminophénylalanine (R,S). Au terme de 40 heures de culture, les 1,5 litres de moût issus de 50 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant la 4ζ-éthylamino-dés(4ζ-diméthylamino) pristinamycine I_{A} sont regroupées et évaporées. Le résidu sec est repris par 7 ml d'un mélange 65 % eau et 35 % acétonitrile et injecté sur une colonne semi-préparative Nucléosil 7µ C8 10x250 mm (Macherey Nagel) éluée dans un mélange de 60 % de tampon phosphate 100 mM pH 2,9 et 40 % d'acétonitrile. Les fractions contenant la 4ζ-éthylamino-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 10 mg de 4ζ-éthylamino-dés(4ζ-diméthylamino) pristinamycine I_{A}.

Spectre de R.M.N. ¹ H (400 MHz, CDCl₃, δ en ppm, ref. TMS)

0,72 (dd, J = 16 et 6 Hz, 1H : 1H du CH₂ en 5 β) ; 0,90 (t, J = 7,5 Hz, 3H : CH₃ en 2 γ) ; 1,15 (mt, 1H : 1H du CH₂ en 3 β) ; de 1,20 à 1,40 (mt, 1H : 1H du CH₂ en 3 γ) ; 1,27 (t, J = 7,5 Hz, 3H : CH₃ de l'éthyle) ; 1,33 (d, J = 7 Hz, 3H : CH₃ en 1 γ) ; de 1,50 à 1,65 (mt, 1H : l'autre H du CH₂ en 3 γ) ; 1,60 et 1,74 (2 mts, 1H chacun : CH₂ en 2 β) ; 2,02 (mt, 1H : l'autre H du CH₂ en 3 β) ; 2,21 et 2,33 (respectivement mt et d large, J = 16,5 Hz, 1H chacun : CH₂ en 5 δ) ; 2,40 (d, J = 16 Hz, 1H : l'autre H du CH₂ en 5 β) ; 2,82 (dt, J = 13 et 4,5 Hz, 1H : 1H du CH₂ en 5 ε) ; 2,89 (dd, J = 12 et 4 Hz, 1H : 1H du CH₂ en 4 β) ; 3,10 (mt, 2H : NCH₂ de l'éthyle) ; de 3,20 à 3,35 (mt, 1H : 1H du CH₂ en 3 δ) ; 3,26 (s, 3H : NCH₃) ; 3,31 (t, J = 12 Hz, 1H : l'autre H du CH₂ en 4 β) ; 3,54 (mt, 1H : l'autre H du CH₂ en 3 δ) ; 3,67 (mf, 1H : NH) ; 4,56 (dd, J = 6,5 et 7 Hz, 1H : 3 α) ; 4,75 (dd large, J = 13 et 8 Hz, 1H : l'autre H du CH₂ en 5 ε) ; 4,84 (mt, 1H : 2 α) ; 4,90 (d large, J = 10 Hz, 1H : 1 α) ; 5,24 (dd, J = 12 et 4 Hz, 1H : 4 α) ; 5,32 (d large, J = 6 Hz, 1H : 5 α) ; 5,88 (d, J = 9,5 Hz, 1H : 6 α) ; 5,90 (mt, 1H : 1 β) ; 6,52 (d, J = 8 Hz, 3H : NH en 2 et H Aromatiques en 4 ε) ; 7,00 (d, J = 8 Hz, 2H : H Aromatiques en 4 δ) ; de 7,10 à 7,35 (mt, 5H : H Aromatiques en 6) ; 7,46 (AB limite, 2H : 1' H₄ et 1' H₅) ; 7,84 (dd, J = 4 et 1 Hz, 1H : 1' H₆) ; 8,45 (d, J = 10 Hz, 1H : NH en 1) ; 8,77 (d, J = 9,5 Hz, 1H : NH en 6) ; 11,65 (s, 1H : OH).

Le dichlorhydrate de 4-éthylaminophénylalanine (R,S) peut être préparé selon la méthode décrite par F. Bergel, J.A. Stock, J. Chem. Soc, 90-97 (1959).

### Exemple S

### Préparation de la 4ζ-trifluorométhoxy dés(4ζ-diméthylamino) pristinamycine I_{A}

On réalise à l'échelle de 60 erlenmeyers comme décrit dans l'exemple F une culture de la souche SP92::pVRC508 en milieu de production avec ajout à 16 heures de 1 ml d'une solution à 20 g/l dans l'eau de chlorhydrate de 4-O-trifluorométhyltyrosine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par du dichlorométhane et injecté sur une colonne de silice (30 g) montée dans le dichlorométhane et éluée successivement par paliers de 0 à 10 % de méthanol dans le dichlorométhane. Les fractions contenant la 4ζ-trifluorométhoxy-dés(4ζ-diméthylamino) pristinamycine I_{A} sont regroupées et évaporées. Le résidu sec est repris par 7 ml d'un mélange 60 % eau et 40 % acétonitrile et injecté sur une colonne semi-préparative Nucléosil 7µ C8 10x250 mm (Macherey Nagel) éluée dans un mélange de 60 % de tampon phosphate 100 mM pH 2,9 et 40 % d'acétonitrile. Les fractions contenant la 4ζ-trifluorométhoxy-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 46,5 mg de 4ζ-trifluorométhoxy-dés(4ζ-diméthylamino) pristinamycine I_{A}.

Spectre de R.M.N. ¹ H (400 MHz, CDCl₃, δ en ppm, ref. TMS)

0,77 (dd, J = 16 et 5,5 Hz, 1H : 1H du CH₂ en 5 β) ; 0,92 (t, J = 7,5 Hz, 3H : CH₃ en 2 γ) ; 1,08 (mt, 1H : 1H du CH₂ en 3 β) ; de 1,30 à 1,40 (mt, 1H : 1H du CH₂ en 3 γ) ; 1,33 (d, J = 7 Hz, 3H : CH₃ en 1 γ) ; de 1,55 à 1,70 (mt, 1H : l'autre H du CH₂ en 3 γ) ; 1,65 et 1,76 (2 mts, 1H chacun : CH₂ en 2 β) ; 2,02 (mt, 1H : l'autre H du CH₂ en 3 β) ; 2,11 et 2,40 (respectivement mt et d large, J = 16,5 Hz, 1H chacun : CH₂ en 5 δ) ; 2,54 (d, J = 16 Hz, 1H : l'autre H du CH₂ en 5 β) ; 2,88 (dt, J = 13 et 4 Hz, 1H : 1H du CH₂ en 5 ε) ; 3,08 (dd, J = 12 et 5 Hz, 1H : 1H du CH₂ en 4 β) ; 3,22 (s, 3H : NCH₃) ; de 3,30 à 3,45 (mt, 1H : 1H du CH₂ en 3 δ) ; 3,39 (t, J = 12 Hz, 1H : l'autre H du CH₂ en 4 β) ; 3,59 (mt, 1H : l'autre H du CH₂ en 3 δ) ; 4,53 (t, J = 7,5 Hz, 1H : 3 α) ; 4,75 (dd large, J = 13 et 8 Hz, 1H : l'autre H du CH₂ en 5 ε) ; 4,85 (mt, 1H : 2 α) ; 4,89 (dd, J = 10 et 1,5 Hz, 1H : 1 α) ; 5,35 (d large, J = 5,5 Hz, 1H : 5 α) ; 5,41 (dd, J = 12 et 5 Hz, 1H : 4 α) ; 5,92 (d, J = 10 Hz, 1H : 6 α) ; 5,93 (mt, 1H : 1 β) ; 6,53 (d, J = 9,5 Hz, 1H : NH en 2) ; de 7,15 à 7,35 (mt, 5H : H Aromatiques en 6) ; 7,16 (d, J = 8 Hz, 2H : H Aromatiques en 4 ε) ; 7,26 (d, J = 8 Hz, 2H : H Aromatiques en 4 δ) ; 7,37 (dd, J = 8,5 et 4 Hz, 1H : 1' H₅) ; 7,42 (dd, J= 8,5 et 1,5 Hz, 1H : 1' H₄) ; 7,70 (dd, J = 4 et 1,5 Hz, 1H : 1' H₆) ; 8,37 (d, J = 10 Hz, 1H : NH en 1); 8,75 (d, J = 10 Hz, 1H : NH en 6) ; 11,66 (s, 1H : OH).

Le chlorhydrate de 4-O-trifluorométhyltyrosine (R,S) peut être préparé de la manière suivante :

En opérant comme à l'exemple N mais à partir de 3 g de N-acétyl (4-trifluorométhoxy) phénylalaninate de méthyle et de 30 ml d'acide chlorhydrique 12N, on obtient 1,5 g de chlorhydrate de 4-O-trifluorométhyltyrosine (RS) sous forme de cristaux blancs fondant à 260°C.

Le N-acétyl (4-trifluorométhoxy) phénylalaninate de méthyle (R,S) peut être préparé de la manière suivante :

En opérant comme à l'exemple N, mais à partir de 3,1 g de (4-trifluorométhoxy) 2-acétamido cinnamate de méthyle, de 0,3 g de palladium sur charbon à 10 % dans 50 ml d'éthanol, on obtient 3 g de N-acétyl (4-trifluorométhoxy) phénylalaninate de méthyle sous forme d'un solide blanc fondant à 80°C.

Le 4-trifluorométhoxy 2-acétamido cinnamate de méthyle peut être préparé de la manière suivante :

En opérant comme à l'exemple N, mais à partir de 4,3 g de 2-acétamido acrylate de méthyle, 0,14 g d'acétate de palladium, 6,1 g de chlorure de tétrabutyl ammonium, de 4,6 g d'hydrogénocarbonate de sodium et de 5 g de 4-trifluorométhoxy bromo benzène en solution dans 150 ml de diméthylformamide anhydre. On obtient 3,1 g de (4-trifluorométhoxy) 2-acétamido cinnamate de méthyle sous forme d'un solide blanc fondant à 135°C.

### Exemple T

### Préparation de la 4ε-méthylthio-dés(4ζ-diméthylamino) pristinamycine I_{A}.

On réalise à l'échelle de 56 erlenmeyers comme décrit dans l'exemple F une culture de la souche SP92::pVRC508 en milieu de production avec ajout à 16 heures de 1 ml d'une solution à 20 g/l dans la soude 0,1N de chlorhydrate de 3-méthylthiophénylalanine (R,S). Au terme de 40 heures de culture, les 1,68 litres de moût issus de 56 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 7 ml d'un mélange 54 % eau et 46 % acétonitrile et injecté sur une colonne semi-préparative Nucléosil 7µ C8 10x250 mm (Macherey Nagel) éluée dans un mélange de 55 % de tampon phosphate 100 mM pH 2,9 et 45 % d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 20 mg de 4ε-méthylthio-dés (4ζ-diméthylamino) pristinamycine I_{A}.

Spectre de R.M.N. ¹ H (400 MHz, CDCl₃, δ en ppm, ref. TMS)

0,56 (dd, J = 16 et 5,5 Hz, 1H : 1H du CH₂ en 5 β) ; 0,90 (t, J = 7,5 Hz, 3H : CH₃ en 2 γ) ; 1,13 (mt, 1H : 1H du CH₂ en 3 β) ; 1,28 (mt, 1H : 1H du CH₂ en 3 γ) ; 1,32 (d, J = 6,5 Hz, 3H : CH₃ en 1 γ) ; 1,58 (mt, 1H : l'autre H du CH₂ en 3 γ) ; 1,62 et 1,74 (2 mts, 1H chacun : CH₂ en 2 β) ; 2,02 (mt, 1H : l'autre H du CH₂ en 3 β) ; 2,25 et 2,35 (respectivement mt et d large, J = 16,5 Hz, 1H chacun : CH₂ en 5 δ) ; 2,39 (d, J = 16 Hz, 1H : l'autre H du CH₂ en 5 β) ; 2,43 (s, 3H : SCH₃) ; 2,82 (dt, J = 13 et 4 Hz, 1H : 1H du CH₂ en 5 ε) ; 2,98 (dd, J = 12 et 4,5 Hz, 1H : 1H du CH₂ en 4 β) ; 3,26 (s, 3H : NCH₃) ; 3,30 (t, J = 12 Hz 1H : 1H du CH₂ en 3 δ) ; 3,38 (mt, 1H : l'autre H du CH₂ en 4 β) ; 3,57 (mt, 1H : l'autre H du CH₂ en 3 δ) ; 4,56 (t, J = 7,5 Hz, 1H : 3 α) ; 4,74 (dd large, J = 13 et 8 Hz, 1H : l'autre H du CH₂ en 5 ε) ; 4,84 (mt, 1H : 2 α) ; 4,89 (dd, J = 10 et 1 Hz, 1H : 1 α) ; 5,29 (dd, J = 12 et 4,5 Hz, 1H : 4 α) ; 5,32 (d large, J = 5,5 Hz, 1H : 5 α) ; 5,88 (d, J = 9,5 Hz, 1H : 6 α) ; 5,90 (mt, 1H : 1 β) ; 6,51 (d, J = 10 Hz, 1H : NH en 2) ; 6,99 (d large, J = 8 Hz, 1H : H Aromatique en para du méthylthio) ; 7,10 et 7,15 (respectivement s large et d large, J = 8 Hz, 1H chacun : H Aromatiques en ortho du méthylthio) ; de 7,15 à 7,35 (mt, 6H : H Aromatiques en 6 et H Aromatiques en méta du méthylthio) ; 7,43 (d large, J = 8 Hz, 1H : 1' H₄) ; 7,52 (dd, J = 8 et 4 Hz, 1H : 1' H₅) ; 7,79 (d large, J = 4 Hz, 1H : 1' H₆) ; 8,38 (d, J = 10 Hz, 1H : NH en 1) ; 8,73 (d, J = 9,5 Hz, 1H : NH en 6) ; 11,62 (s, 1H : OH).

Le chlorhydrate de 3-méthylthiophénylalanine (R,S) peut être préparé de la manière suivante :

En opérant comme à l'exemple N, mais à partir de 3,3 g de N-acétyl 3-méthylthio phénylalaninate de méthyle et de 40 ml d'acide chlorhydrique 12N, on obtient 1,38 g de chlorhydrate de 3-méthylthio phénylalanine (RS) sous forme de cristaux blancs fondant à 190°C.

Le N-acétyl 3-méthylthio phénylalaninate de méthyle (RS) peut être préparé de la manière suivante :

On place dans un ballon 3,72 g de 3-méthylthio 2-acétamido cinnamate de méthyle en solution dans 100 ml de méthanol et 30 ml de tétrahydrofuranne puis on ajoute 1,4 g de magnésium. Après 20 minutes de réaction on refroidit le mélange par un bain de glace puis on ajoute à nouveau 1,4 g de magnésium. Le mélange est agité à température ambiante pendant 18 heures, versé sur 1,4 1 d'eau distillée et 300 ml de CH₂Cl₂ puis filtré sur Clarcel®. La phase aqueuse est ajustée à pH 6 par addition d'acide chlorhydrique 12 N puis décantée et lavée par 100 ml de CH₂Cl₂. Les phases organiques sont rassemblées, séchées sur sulfate de magnésium, filtrées puis concentrées à sec sous pression réduite pour donner 3,42 g de N-acétyl 3-méthylthio phénylalaninate de méthyle sous forme d'une huile incolore (Silice Merck 5719, Rf= 0,5 ; AcOEt).

Le 3-méthylthio 2-acétamido cinnamate de méthyle peut être préparé de la manière suivante :

En opérant comme à l'exemple N, mais à partir de 5,6 g de 2-acétamido acrylate de méthyle, 0,18 g d'acétate de palladium, 8,2 g de chlorure de tétrabutyl ammonium, de 5,86 g d'hydrogénocarbonate de sodium et de 6,5 g de 3-iodo 1-méthylthiobenzène en solution dans 160 ml de diméthylformamide anhydre, on obtient 4,8 g de (3-méthylthio) 2-acétamido cinnamate de méthyle sous forme d'un solide blanc fondant à 139°C.

### Exemple U

### Préparation de la 4ζ-(allyl éthyl amino) dés(4ζ-diméthylamino) pristinamycine I_{A}

On réalise à l'échelle de 26 erlenmeyers comme décrit dans l'exemple F une culture de la souche SP92::pVRC508 en milieu de production avec ajout à 16 heures de 1 ml d'une solution à 20 g/l dans la soude 0,1N de dichlorhydrate de 4-(allyl éthyl amino)phényl alanine (R,S). Au terme de 40 heures de culture, les 0,78 litre de moût issus de 26 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant la 4ζ-(allyl éthyl amino) dés(4ζ-diméthylamino) pristinamycine I_{A} sont regroupées et évaporées. Le résidu sec est repris par 7 ml d'un mélange 60 % eau et 40 % acétonitrile et injecté sur une colonne semi-préparative Nucléosil 7µ C8 10x250 mm (Macherey Nagel) éluée dans un mélange de 52 % de tampon phosphate 100 mM pH 2,9 et 48 % d'acétonitrile. Les fractions contenant la 4ζ-(allyl éthyl amino) dés(4ζ-diméthylamino) pristinamycine I_{A} sont regroupées et extraites par un volume de dichlorométhane. La phase organique est lavée à l'eau, séchée sur sulfate de sodium puis évaporée. On obtient 20 mg de 4ζ-(allyl éthyl amino) dés(4ζ-diméthylamino) pristinamycine I_{A}.

Spectre de R.M.N. ¹ H (400 MHz, CDCl₃, δ en ppm, ref. TMS)

0,58 (dd, J = 16 et 6 Hz, 1H : 1H du CH₂ en 5 β) ; 0,91 (t, J = 7,5 Hz, 3H : CH₃ en 2 γ) ; 1,16 (t, J = 7 Hz, 3H : CH₃ de l'éthyle) ; 1,16 (mt, 1H : 1H du CH₂ en 3 β) ; 1,25 (mt, 1H : 1H du CH₂ en 3 γ) ; 1,32 (d, J = 6,5 Hz, 3H : CH₃ en 1 γ) ; 1,54 (mt, 1H : l'autre H du CH₂ en 3 γ) ; 1,63 et 1,75 (2 mts, 1H chacun : CH₂ en 2 β) ; 2,02 (mt, 1H : l'autre H du CH₂ en 3 β) ; 2,23 et 2,31 (respectivement mt et d large, J = 16,5 Hz, 1H chacun : CH₂ en 5 δ) ; 2,37 (d, J = 16 Hz, 1H : l'autre H du CH₂ en 5 β) ; 2,80 (dt, J = 13 et 4,5 Hz, 1H : 1H du CH₂ en 5 ε) ; 2,87 (dd, J = 12 et 4 Hz, 1H : 1H du CH₂ en 4 β) ; de 3,15 à 3,30 (mt, 1H : 1H du CH₂ en 3 δ) ; 3,26 (s, 3H : NCH₃) ; 3,30 (t, J = 12 Hz, 1H : l'autre H du CH₂ en 4 β) ; 3,36 (mt, 2H : NCH₂ de l'éthyle) ; 3,54 (mt, 1H : l'autre H du CH₂ en 3 δ) ; 3,90 (AB limite, 2H : NCH₂ de l'allyle) ; 4,57 (dd, J = 8 et 6 Hz, 1H : 3 α) ; 4,76 (dd large, J = 13 et 7,5 Hz, 1H : l'autre H du CH₂ en 5 ε) ; 4,84 (mt, 1H : 2 α) ; 4,89 (dd, J = 10 et 1 Hz, 1H : 1 α) ; de 5,05 à 5,20 (mt, 3H : 4 α et =CH₂ de l'allyle) ; 5,27 (d large, J = 6 Hz, 1H : 5 α) ; 5,83 (mt, 1H : CH= de l'allyle) ; 5,88 (d, J = 9,5 Hz, 1H : 6 α) ; 5,91 (mt, 1H : 1 β) ; 6,50 (d, J = 10 Hz, 1H : NH en 2) ; 6,60 (d, J = 8 Hz, 2H : H Aromatiques en 4 ε) ; 7,02 (d, J = 8 Hz, 2H : H Aromatiques en 4 δ) ; de 7,10 à 7,35 (mt, 5H : H Aromatiques en 6) ; 7,47 (AB limite, 2H : 1' H₄ et 1' H₅) ; 7,88 (dd, J = 4 et 2 Hz, 1H : 1' H₆) ; 8,41 (d, J = 10 Hz, 1H : NH en 1) ; 8,75 (d, J = 9,5 Hz, 1H : NH en 6) ; 11,62 (s, 1H : OH).

Le dichlorhydrate de 4-(allyl éthyl amino)phényl alanine (R,S) peut être préparé de la manière suivante :

En opérant comme à l'exemple F mais à partir de 4,54 g de 4-allyl éthylbenzyl acétamido malonate de diéthyle et de 37,9 ml d'acide chlorhydrique à 10N, on obtient après évaporation un solide qui est séché sous pression réduite (2,7 kPa) à 40°C. On obtient 3,67 g de dichlorhydrate de 4-(allyl éthyl amino)phényl alanine (RS) sous forme d'un solide brun fondant vers 130°C (décomposition).

Le 4-(allyl éthyl amino)benzyl acétamido malonate de diéthyle peut être préparé de la manière suivante :

En opérant comme à l'exemple F mais à partir de 8 g de 4-éthylaminobenzylacétamido malonate de diéthyle, 4 ml de bromure d'allyle, 5,82 ml de 1,5-diazabicyclo[4-3-0]non-5-ène, dans 50 ml de tétrahydrofurane, on obtient après purification par chromatographie flash (éluant CH₂Cl₂ / AcOEt 90-10 en volume) 5,6 g d'un solide qui est recristallisé dans 35 ml de cyclohexane. On obtient ainsi 5,43 g de 4-(allyl éthyl amino)benzyl acétamido malonate de diéthyle sous forme d'un solide blanc fondant à 86°C.

### Exemple V

### Préparation de la 4ζ-(éthyl propyl amino) dés(4ζ-diméthylamino) pristinamycine I_{A}

On réalise à l'échelle de 60 erlenmeyers comme décrit dans l'exemple F une culture de la souche SP92::pVRC508 en milieu de production avec ajout à 16 heures de 1 ml d'une solution à 20 g/l dans la soude 0,1N de dichlorhydrate de 4-(éthyl propyl amino)phényl alanine (R,S). Au terme de 40 heures de culture, les 1,8 litres de moût issus de 60 erlenmeyers sont extraits par 2 volumes d'un mélange de 66 % de tampon phosphate 100 mM pH 2,9 et 34 % d'acétonitrile, puis centrifugés. Le surnageant est extrait par 2 fois 0,5 volume de dichlorométhane. Les phases chlorométhyléniques sont lavées à l'eau puis combinées, séchées sur sulfate de sodium et évaporées. L'extrait sec est repris par 20 ml de dichlorométhane et injecté sur une colonne de silice (30 g) montée dans le dichlorométhane et éluée successivement par paliers de 0 à 10 % de méthanol dans le dichlorométhane. Les fractions contenant la 4ζ-(éthyl propyl amino) dés (4ζ-diméthylamino) pristinamycine I_{A} sont regroupées et évaporées. Le résidu sec est repris par 7 ml d'un mélange 60 % eau et 40 % acétonitrile et injecté sur une colonne semi-préparative Nucléosil 7µ C8 10x250 mm (Macherey Nagel) éluée dans un mélange de 63 % de tampon phosphate 100 mM pH 2,9 et 37 % d'acétonitrile. Les fractions contenant la 4ζ-(éthyl propyl amino) dés(4ζ-diméthylamino) pristinamycine I_{A} sont regroupées et extraites par un volume de dichlorométhane. La phase organique est lavée à l'eau, séchée sur sulfate de sodium puis évaporée. On obtient 16 mg de 4ζ-(éthyl propyl amino) dés(4ζ-diméthylamino) pristinamycine I_{A}.

Spectre de R.M.N. ¹ H (400 MHz, CDCl₃, δ en ppm, ref. TMS)

0,67 (dd, J = 16 et 6 Hz, 1H : 1H du CH₂ en 5 β) ; 0,91 (t, J = 7,5 Hz, 3H : CH₃ en 2 γ) ; 0,95 (t, J = 7,5 Hz, 3H : CH₃ du propyle) ; 1,14 (t, J = 7 Hz, 3H : CH₃ de l'éthyle) ; 1,15 (mt, 1H : 1H du CH₂ en 3 β) ; 1,25 (mt, 1H : 1H du CH₂ en 3 γ) ; 1,33 (d, J = 7 Hz, 3H : CH₃ en 1 γ) ; de 1,45 à 1,65 (mt, 3H : l'autre H du CH₂ en 3 γ et CH₂ propyle) ; 1,63 et 1,75 (2 mts, 1H chacun : CH₂ en 2 β) ; 2,02 (mt, 1H : l'autre H du CH₂ en 3 β) ; 2,23 et 2,33 (respectivement mt et d large, J = 16,5 Hz, 1H chacun : CH₂ en 5 δ) ; 2,37 (d, J = 16 Hz, 1H : l'autre H du CH₂ en 5 β) ; 2,80 (dt, J = 13 et 5 Hz, 1H : 1H du CH₂ en 5 ε) ; 2,89 (dd, J = 12 et 4 Hz, 1H : 1H du CH₂ en 4 β) ; de 3,10 à 3,25 (mt, 3H : 1H du CH₂ en 3 δ et NCH₂ du propyle) ; 3,26 (s, 3H : NCH₃) ; de 3,25 à 3,40 (mt, 2H : NCH₂ de l'éthyle) ; 3,34 (t, J= 12 Hz, 1H : l'autre H du CH₂ en 4 β) ; 3,54 (mt, 1H : l'autre H du CH₂ en 3 δ) ; 4,57 (dd, J = 7,5 et 6 Hz, 1H : 3 α) ; 4,76 (dd large, J = 13 et 7,5 Hz, 1H : l'autre H du CH₂ en 5 ε) ; 4,84 (mt, 1H : 2 α) ; 4,89 (dd, J = 10 et 1 Hz, 1H : 1 α) ; 5,21 (dd, J = 12 et 4 Hz, 1H : 4 α) ; 5,28 (d large, J = 6 Hz, 1H : 5 α) ; 5,88 (d, J = 9,5 Hz, 1H : 6 α) ; 5,91 (mt, 1H : 1 β) ; 6,48 (d, J = 10 Hz, 1H : NH en 2) ; 6,60 (d, J = 8 Hz, 2H : H Aromatiques en 4 ε) ; 7,03 (d, J = 8 Hz, 2H : H Aromatiques en 4 δ) ; de 7,10 à 7,35 (mt, 5H : H Aromatiques en 6) ; 7,47 (AB limite, 2H : 1' H₄ et 1' H₅) ; 7,89 (mt, 1H : 1' H₆) ; 8,42 (d, J = 10 Hz, 1H : NH en 1) ; 8,76 (d, J = 9,5 Hz, 1H : NH en 6) ; 11,62 (s, 1H : OH).

Le dichlorhydrate de 4-(éthyl propyl amino)phényl alanine (R,S) peut être préparé de la manière suivante :

En opérant comme à l'exemple F mais à partir de 2,5 g de 4-éthyl propylaminobenzyl acétamido malonate de diéthyle et de 21 ml d'acide chlorhydrique à 10N, on obtient après évaporation un solide qui est séché sous pression réduite (2,7 kPa) à 40°C. On obtient 2 g (97 %) de dichlorhydrate de 4-(éthyl propyl amino)phényl alanine (RS) sous forme d'un solide blanc fondant vers 147°C (décomposition).

Le 4-(éthyl propyl amino)benzyl acétamido malonate de diéthyle peut être préparé de la manière suivante :

En opérant comme à l'exemple F mais à partir de 10 g de 4-éthylaminobenzylacétamido malonate de diéthyle, 5,6 ml de 1-iodo propane, 7,2 ml de 1,5-diazabicyclo[4-3-0]non-5-ène, dans 70 ml de tétrahydrofurane, on obtient après 36 heures de réaction puis purification par chromatographie flash (éluant CH₂Cl₂ / MeOH 97-3 en volume), 2,8 g d'un solide qui est recristallisé dans 26 ml de cyclohexane. On obtient ainsi 2,9 g de 4-(éthyl propyl amino)benzyl acétamido malonate de diéthyle sous forme d'un solide blanc fondant à 84-86°C.

### SEPARATION ET PURIFICATION DE COMPOSANTES DU GROUPE B :

### Exemple W

30 kg de pristinamycine brute [pristinamycine IA (PIA) : 20,7 %, pristinamycine IB (PIB) : 3,9 %, pristinamycine IC (PIC) : 0,6 %, pristinamycine ID (PID) : 0,3 %, pristinamycine IIB (PIIB) : 8 %, pristinamycine IIA (PIIA) : 45 %, pristinamycine IIF (PIIF) : < 0,5 % (non dosé), pristinamycine IIG (PIIG) : < 0,5 % (non dosé)] sont mis en suspension dans 210 litres d'acétate d'éthyle et agités pendant 15 heures à température ambiante. La suspension est filtrée et le filtrat acétate d'éthyle recueilli est extrait par 2 fois 20 litres d'acide sulfurique 1N puis 20 litres d'eau distillée. Les phases aqueuses réunies sont lavées par 6 fois 15 litres d'acétate d'éthyle, puis ajustées à pH 7 par addition de 30 litres d'une solution à 10 % de bicarbonate de sodium et extraites par 3 fois 30 litres de chlorure de méthylène. Les phases chlorométhyléniques sont réunies et lavées par 10 litres d'eau distillée. Le chlorure de méthylène est ensuite distillé et remplacé par 50 litres d'éthanol. Le mélange est alors traité au reflux par 0,8 kg de noir L3S pendant 30 minutes. Après filtration et lavage par 2 fois 5 litres d'éthanol, le mélange est refroidi jusqu'à 10°C en 15 heures. Après maintien une heure à 10°C, la suspension est filtrée et lavée par 3 fois 7 litres d'éthanol. Après séchage du solide à 40°C sous pression réduite, on obtient 5,7 kg de pristinamycine I purifiée (ci-après appelée PI).

Titre: 96,8 % (PIA : 81,1 %, PIB : 12 %, PIC : 2,6 %, PID : 1,1 %);

Rendement en PIA : 74 %.

1500 g de PI purifiée sont repris par 9 litres de dichloro-1,2 éthane puis additionnés de 1,5 équivalent d'anhydride succinique et de 0,015 équivalent de diméthylaminopyridine. La solution est maintenue 1 semaine à 20°C, puis introduite sur une colonne contenant 10 kg de silice (20-45 µm) [hauteur de la colonne : 1 m ; diamètre : 20 cm]. L'élution est effectuée par percolation d'un mélange dichloro-1,2 éthane/méthanol à un débit de 18 litres/heure pendant 6 heures ; le pourcentage de méthanol (à 5 % de teneur en eau) est augmenté de 0 à 4 % au cours de la chromatographie. 47 fractions de 2,4 litres sont récupérées.

Les fractions 5 à 15 sont réunies, le dichloro-1,2 éthane est évaporé et remplacé par 5 litres d'éthanol. Après cristallisation, on obtient 365 g de PIA titrant 99,8 %.

### PREPARATION DE COMPOSANTES BRUTES DU GROUPE A :

### Exemple X

500 g de pristinamycine brute [pristinamycine IA (PIA) : 20,7 %, pristinamycine IB (PIB) : 3,9 %, pristinamycine IC (PIC) : 0,6 %, pristinamycine ID (PID) : 0,3 %, pristinamycine IIB (PIIB) : 8 %, pristinamycine IIA (PIIA): 45 %] sont mis en solution dans 50 litres de méthylisobutylcétone. Cette solution est extraite 5 fois par une phase aqueuse composée de 2,5 litres d'eau et 2,5 litres d'acide sulfurique 1 N, puis lavée par 3 fois 10 litres d'eau. La méthylisobutylcétone est ensuite traitée par 7,5 litres d'une solution aqueuse d'hydrogénocarbonate de sodium à 35 g/litre, puis lavée par 5 litres d'eau. A chaque fois, la phase aqueuse est mélangée avec la phase organique, décantée et séparée.

La phase organique obtenue est mise en contact avec 750 g d'alumine, filtrée, concentrée jusqu'à un volume de 4 litres environ et reprise par 5 volumes d'hexane. Le précipité obtenu est filtré et séché. On obtient 300 g de produit que l'on met en suspension dans 1 litre d'isopropanol. Après agitation à 55°C pendant 45 minutes, on filtre à 4°C. Les eaux-mères de filtration sont concentrées à sec, reprises par 500 cm³ de méthylisobutylcétone sur lesquels on verse 5 volumes d'hexane. Le précipité est filtré, lavé à l'hexane et séché à 40°C sous pression réduite. On obtient 69 g de PIIB brute contenant 36 % de PIIB, 6 % de PIIA et ne contenant plus de PIA.

### Exemple Y

60 g de PIIB brute obtenue comme précédemment à l'exemple P sont purifiés en plusieurs opérations, par chromatographie sur colonne de Nucléosil 5C8® (diamètre de la colonne 5 cm, hauteur 30 cm) percolée par un éluant eau/acétonitrile 60/40. On obtient ainsi 250 mg de pristinamycine IIF (PIIF).

La présente invention concerne également les compositions pharmaceutiques utilisables en médecine humaine ou vétérinaire qui contiennent comme produit actif la nouvelle association purifiée de streptogramine comprenant au moins une composante du groupe B des streptogramines définie par la formule générale (I) cocristallisée avec au moins une composante du groupe A de formule générale (II), à l'état pur ou en présence d'un ou plusieurs diluants ou adjuvants compatibles et pharmaceutiquement acceptables. Ces compositions peuvent être utilisées par voie orale ou topique.

Comme compositions pour administration orale, peuvent être utilisés des comprimés, des gélules, des pilules, des poudres des lyophilisats ou des granulés. Dans ces compositions, le produit actif selon l'invention peut être mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Les compositions pour administration topique peuvent être par exemple des crèmes, des pommades, ou des lotions.

En thérapeutique humaine ou vétérinaire, les compositions selon l'invention sont particulièrement utiles dans le traitement des infections d'origine bactérienne notamment les infections graves à cocci Gram-positif : infections à staphylocoques (notamment les infections à staphylocoque résistant à la méthicilline), infections à streptocoques (notamment sur les pneumocoques résistants à la pénicilline et aux macrolides) ; ils sont aussi particulièrement utiles dans le traitement des infections à Hemophilus, Moraxella catarrhalis, Neisseria gonorrhoeae, Chlamydia trachomatis, Mycoplasma hominis, Mycoplasma pneumoniae, Ureaplasma urealyticum.

Les compositions selon l'invention peuvent être employées notamment dans le traitement des infections respiratoires hautes et basses (par exemple traitement des infections pulmonaires), dans le traitement des infections cutanées, dans le traitement à long terme des infections osseuses ou articulaires, dans le traitement ou la prophylaxie de l'endocardite en chirurgie dentaire et urinaire, dans le traitement des maladies sexuellement transmisibles, ainsi que dans le traitement des infections opportunistes bactériennes et parasitaires survenant dans le SIDA et en prophylaxie du risque staphylococcique chez l'immunodéprimé.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 0,4 et 3,5 g de produit actif en 2 ou 3 prises par jour, par voie orale pour un adulte.

Les exemples suivants, donnés à titre non limitatif, illustrent des compositions selon l'invention :

### Exemple A

On prépare selon les techniques habituelles des gélules opaques dosées à 250 mg de l'association cocristallisée 4ε-chloro pristinamycine I_{A}/pristinamycine IIB.

### Exemple B

On prépare selon les techniques habituelles des gélules opaques dosées à 250 mg de l'association cocristallisée dés(4ζ-diméthylamino) 4ε-méthoxy pristinamycine I_{A}/pristinamycine IIB.

## Revendications

1. Une forme purifiée de streptogramines caractérisée en ce qu'elle comprend au moins une composante du groupe B des streptogramines définie par la formule générale : 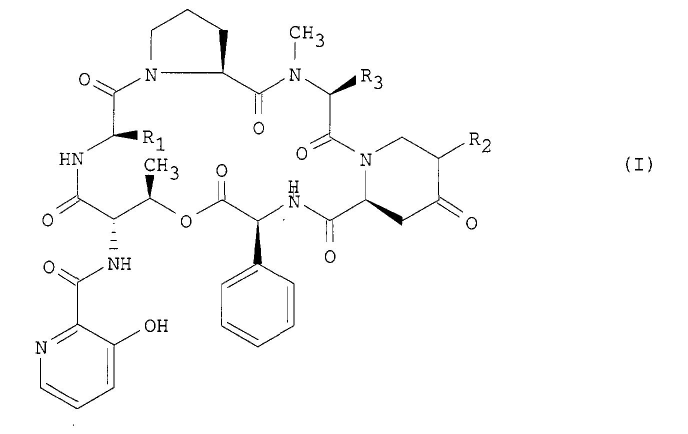 dans laquelle
- le symbole R₁ représente un radical méthyle ou éthyle, et
1) soit
- le symbole R₂ représente un atome d'hydrogène et
- le symbole R₃ représente un radical benzyle substitué de formule générale : 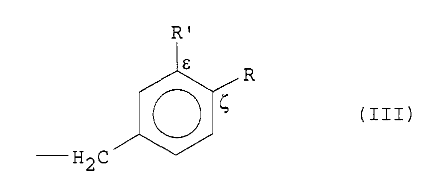 dans laquelle R représente NR₄R₅ pour lequel les symboles R₄ et R₅ sont l'un un atome d'hydrogène ou un radical méthyle et l'autre un radical méthyle et R' est un atome de chlore ou de brome, ou représente un radical alcényle contenant 3 à 5 atomes de carbone si R₄ et R₅ sont des radicaux méthyle,
2) soit
- le symbole R₂ représente un atome d'hydrogène ou un radical hydroxy, et
- le symbole R₃ représente un radical de formule générale (III)
dans laquelle R est un atome d'hydrogène et R' représente un atome d'halogène, un radical alcoylamino ou dialcoylamino, le reste d'un éther-oxyde, un radical alcoylthio, alcoyle contenant 1 à 3 atomes de carbone en chaîne droite ou ramifiée ou trihalogénométhyle, ou bien
dans laquelle R est un atome d'halogène, un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical alcényl alcoyl amino dont la partie alcényle contient 3 ou 4 atomes de carbone et la partie alcoyle est définie comme ci-dessus, un radical dialcénylamino dont les parties alcényle sont définies comme ci-dessus, un radical alcoyl cycloalcoylméthyl amino dont la partie alcoyle est définie comme ci-dessus et la partie cycloalcoyle contient 3 ou 4 atomes de carbone, le reste d'un éther-oxyde, un radical alcoylthio, un radical alcoylthiométhyle, alcoyle contennant 1 à 6 atomes de carbone en chaîne droite ou ramifiée, aryle ou trihalogénométhyle, et R' est un atome d'hydrogène, ou bien
dans laquelle R est un atome d'halogène, un radical amino, alcoylamino ou dialcoylamino, le reste d'un éther-oxyde, un radical alcoylthio, alcoyle contenant 1 à 6 atomes de carbone en chaîne droite ou ramifiée ou trihalogénométhyle et R' représente un atome d'halogène, un radical alcoylamino ou dialcoylamino, le reste d'un éther-oxyde ou un radical alcoylthio ou alcoyle contenant 1 à 3 atomes de carbone en chaîne droite ou ramifiée,
étant entendu que, sauf mention spéciale, les radicaux alcoyle sont droits ou ramifiés et contiennent 1 à 4 atomes de carbone,
cocristallisée avec au moins une composante "minoritaire" du groupe A des streptogramines, de formule générale : 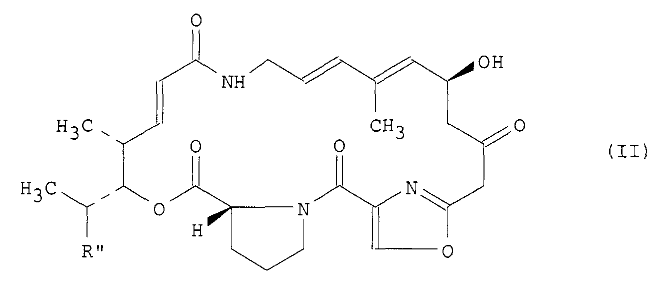 dans laquelle R" est un atome d'hydrogène ou un radical méthyle ou éthyle.

2. Une forme purifiée de streptogramines selon la revendication 1, caractérisée en ce qu'elle comprend au moins une composante du groupe B des streptogramines définie par la formule générale (I) dans la revendication 1, dans laquelle :
le symbole R₁ est défini comme dans la revendication 1, et
1) soit
le symbole R₂ représente un atome d'hydrogène et
le symbole R₃ représente un radical benzyle substitué de formule générale : 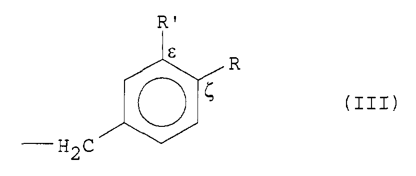 dans laquelle R représente NR₄R₅ pour lequel les symboles R₄ et R₅ sont l'un un atome d'hydrogène ou un radical méthyle et l'autre un radical méthyle et R' est un atome de chlore ou de brome, ou représente un radical alcényle contenant 3 à 5 atomes de carbone si R₄ et R₅ sont des radicaux méthyle,
2) soit
le symbole R₂ représente un atome d'hydrogène ou un radical hydroxy, et
le symbole R₃ représente un radical de formule générale (III)
dans laquelle R est un atome d'hydrogène et R' représente un radical alcoylamino ou dialcoylamino, le reste d'un éther-oxyde ou un radical alcoylthio, ou bien
dans laquelle R est un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical alcényl alcoyl amino dont la partie alcényle contient 3 ou 4 atomes de carbone et la partie alcoyle est définie comme ci-dessus, le reste d'un éther-oxyde, un radical alcoylthio, alcoyle contennant 1 à 6 atomes de carbone en chaîne droite ou ramifiée ou trihalogénométhyle, et R' est un atome d'hydrogène, ou bien
dans laquelle R est un radical alcoyle contenant 1 à 6 atomes de carbone en chaîne droite ou ramifiée et R' représente un atome d'halogène,
étant entendu que, sauf mention spéciale, les radicaux alcoyle sont droits ou ramifiés et contiennent 1 à 4 atomes de carbone,
cocristallisée avec au moins une composante "minoritaire" du groupe A des streptogramines telle que définie dans la revendication 1.

3. Une forme purifiée de streptogramines selon la revendication 1, caractérisée en ce qu'elle comprend au moins une composante du groupe B des streptogramines définie par la formule générale (I) dans la revendication 1, dans laquelle :
le symbole R₁ est un radical éthyle, le symbole R₂ est un atome d'hydrogène, et
le symbole R₃ représente un radical benzyle substitué de formule générale : 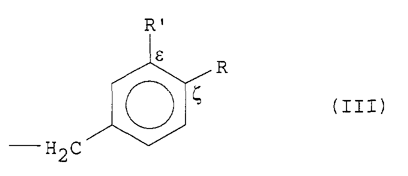 dans laquelle
1) R représente NR₄R₅ pour lequel les symboles R₄ et R₅ sont l'un un atome d'hydrogène ou un radical méthyle et l'autre un radical méthyle et R' est un atome de chlore ou de brome, ou représente un radical allyle si R₄ et R₅ sont des radicaux méthyle, ou bien
2) R est un atome d'hydrogène et R' représente un radical alcoylamino ou dialcoylamino, un radical méthoxy, éthoxy, allyloxy ou trifluorométhoxy, ou un radical alcoylthio, ou bien
R est un radical alcoylamino contenant 2 à 4 atomes de carbone en chaîne droite ou ramifiée, dialcoylamino dont les parties alcoyle sont identiques ou différentes et contiennent 2 à 4 atomes de carbone en chaîne droite ou ramifiée ou méthyl éthyl amino, un radical pyrrolidino, un radical allyl alcoyl amino dont la partie alcoyle est définie comme ci-dessus, un radical méthoxy, éthoxy, allyloxy ou trifluorométhoxy, un radical alcoylthio, alcoyle contennant 1 à 6 atomes de carbone en chaîne droite ou ramifiée ou trifluorométhyle, et R' est un atome d'hydrogène, ou bien
R est un radical alcoyle contenant 1 à 6 atomes de carbone en chaîne droite ou ramifiée et R' représente un atome d'halogène,
étant entendu que, sauf mention spéciale, les radicaux alcoyle sont droits ou ramifiés et contiennent 1 à 4 atomes de carbone,
cocristallisée avec au moins une composante "minoritaire" du groupe A des streptogramines telle que définie dans la revendication 1.

4. Une forme purifiée de streptogramines selon l'une des revendications 1 à 3, caractérisée en ce qu'il s'agit d'un composé cocristallisé d'une ou plusieurs composantes du groupe B des streptogramines définies dans la revendication 1 à 3 et d'une ou plusieurs composantes minoritaires du groupe A des streptogramines telles que définies dans la revendication 1, dans un rapport molaire de l'ordre de 1/2.

5. Un procédé de préparation d'une forme purifiée de streptogramines selon l'une des revendications 1 à 4, caractérisé en ce que l'on cocristallise la(les) composante(s) du groupe A des streptogramines, avec la(les) composantes du groupe B des streptogramines telles que définies dans les revendications 1 à 3.

6. Une composante purifiée du groupe A des streptogramines telle que définie dans la revendication 1, lorsqu'elle est obtenue par l'intermédiaire d'un composé cocristallisé selon la revendication 1 à 4.

7. Utilisation d'une ou plusieurs composantes minoritaires du groupe A des streptogramines telles que définies dans la revendication 1, pour la préparation de cocristallisats avec une ou plusieurs composantes du groupe B des streptogramines définies dans la revendication 1 à 3.

8. Composition pharmaceutique caractérisée en ce qu'elle contient une forme purifiée de streptogramines selon l'une des revendications 1 à 4, à l'état pur ou en présence de tout diluant ou adjuvant compatible et pharmaceutiquement acceptable.

## Patentansprüche

1. Gereinigte Form von Streptograminen, dadurch gekennzeichnet, daß sie mindestens eine Komponente der Gruppe B der Streptogramine umfaßt, definiert durch die allgemeine Formel (I) 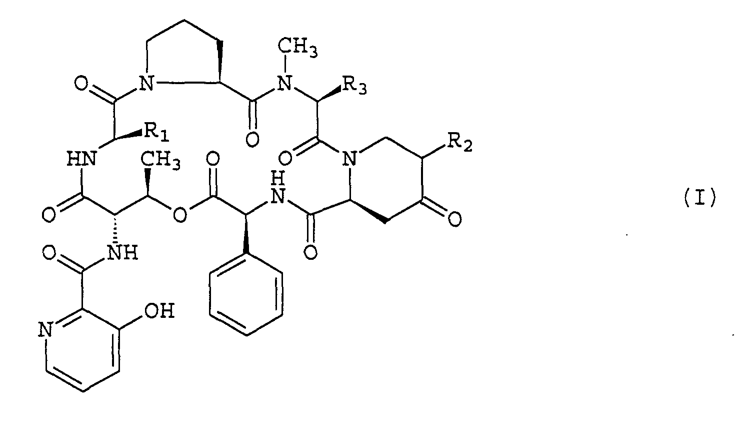 in der
- das Symbol R₁ einen Rest Methyl oder Ethyl darstellt, und
1) entweder
- das Symbol R₂ ein Wasserstoffatom ist und
- das Symbol R₃ einen substituierten Rest Benzyl der allgemeinen Formel (III) bedeutet 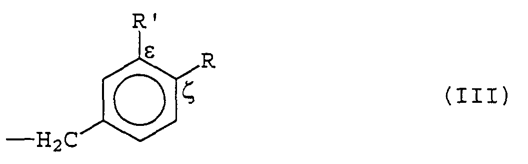 in der R NR₄R₅ darstellt, worin bei den Symbolen R₄ und R₅ das eine ein Wasserstoffatom oder ein Rest Methyl ist und das andere ein Rest Methyl ist und R' ein Chloratom oder Bromatom bedeutet oder einen Rest Alkenyl mit 3 bis 5 Kohlenstoffatomen darstellt, wenn R₄ und R₅ Reste Methyl sind,
2) oder
- das Symbol R₂ ein Wasserstoffatom oder ein Rest Hydroxy ist und
- das Symbol R₃ einen Rest der allgemeinen Formel (III) darstellt, worin R ein Wasserstoffatom ist und R' ein Halogenatom, einen Rest Alkylamino oder Dialkylamino, den Rest eines Ether-oxides, einen Rest Alkylthio, Alkyl mit 1 bis 3 Kohlenstoffatomen in gerader oder verzweigter Kette oder Trihalogenmethyl bedeutet, oder worin R ein Halogenatom, einen Rest Alkylamino mit 2 bis 4 Kohlenstoffatomen in gerader oder verzweigter Kette, Dialkylamino, dessen Teile Alkyl gleich oder verschieden sind und 2 bis 4 Kohlenstoffatome in gerader oder verzweigter Kette enthalten, oder Methyl-ethyl-amino, einen Rest Pyrrolidino, einen Rest Alkenyl-alkyl-amino, dessen Teil Alkenyl 3 oder 4 Kohlenstoffatome enthält und dessen Teil Alkyl wie oben definiert ist, einen Rest Dialkenylamino, dessen Teile Alkenyl wie oben definiert sind, einen Rest Alkyl-cycloalkyl-methyl-amino, dessen Teil Alkyl wie oben definiert ist und dessen Teil Cycloalkyl 3 oder 4 Kohlenstoffatome enthält, den Rest eines Ether-oxides, einen Rest Alkylthio, einen Rest Alkylthiomethyl, Alkyl mit 1 bis 6 Kohlenstoffatomen in gerader oder verzweigter Kette, Aryl oder Trihalogenmethyl darstellt und R' ein Wasserstoffatom ist, oder
worin R ein Halogenatom, einen Rest Amino, Alkylamino oder Dialkylamino, den Rest eines Ether-oxides, einen Rest Alkylthio, Alkyl mit 1 bis 6 Kohlenstoffatomen in gerader oder verzweigter Kette oder Trihalogenmethyl bedeutet, und R' ein Halogenatom, ein Rest Alkylamino oder Dialkylamino, der Rest eines Ether-oxides oder ein Rest Alkylthio oder Alkyl mit 1 bis 3 Kohlenstoffatomen in gerader oder verzweigter Kette ist,
mit der Maßgabe, daß außer bei spezieller Erwähnung die Reste Alkyl gerade oder verzweigt sind und 1 bis 4 Kohlenstoffatome enthalten,
cokristallisiert mit mindestens einer "Minderheits"-Komponente der Gruppe A der Streptogramine der allgemeinen Formel (II) 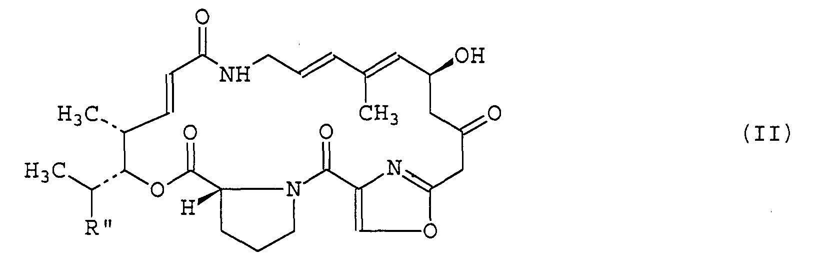 in der R" ein Wasserstoffatom oder ein Rest Methyl oder Ethyl ist.

2. Gereinigte Form von Streptograminen nach Anspruch 1, dadurch gekennzeichnet, daß sie mindestens eine Komponente der Gruppe B der in der allgemeinen Formel (I) in Anspruch 1 definierten Streptogramine umfaßt, in der
- das Symbol R₁ wie in Anspruch 1 definiert ist und
1) entweder
- das Symbol R₂ ein Wasserstoffatom ist und
- das Symbol R₃ einen substituierten Rest Benzyl der allgemeinen Formel (III) bedeutet 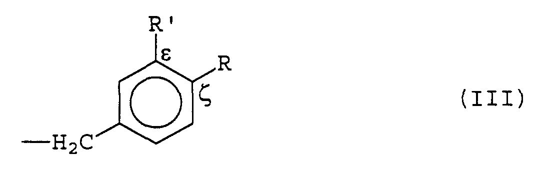 in der R NR₄R₅ darstellt, worin bei den Symbolen R₄ und R₅ das eine ein Wasserstoffatom oder ein Rest Methyl ist und das andere ein Rest Methyl ist und R' ein Chloratom oder Bromatom bedeutet oder einen Rest Alkenyl mit 3 bis 5 Kohlenstoffatomen darstellt, wenn R₄ und R₅ Reste Methyl sind,
2) oder
- das Symbol R₂ ein Wasserstoffatom oder ein Rest Hydroxy ist und
- das Symbol R₃ einen Rest der allgemeinen Formel (III) darstellt, worin R ein Wasserstoffatom ist und R' einen Rest Alkylamino oder Dialkylamino, den Rest eines Ether-oxides oder einen Rest Alkylthio bedeutet, oder
worin R einen Rest Alkylamino mit 2 bis 4 Kohlenstoffatomen in gerader oder verzweigter Kette, Dialkylamino, dessen Teile Alkyl gleich oder verschieden sind und 2 bis 4 Kohlenstoffatome in gerader oder verzweigter Kette enthalten, oder Methyl-ethyl-amino, einen Rest Pyrrolidino, einen Rest Alkenyl-alkyl-amino, dessen Teil Alkenyl 3 oder 4 Kohlenstoffatome enthält und dessen Teil Alkyl wie oben definiert ist, den Rest eines Ether-oxides, einen Rest Alkylthio, Alkyl mit 1 bis 6 Kohlenstoffatomen in gerader oder verzweigter Kette oder Trihalogenmethyl darstellt und R' ein Wasserstoffatom ist, oder
worin R einen Rest Alkyl mit 1 bis 6 Kohlenstoffatomen in gerader oder verzweigter Kette bedeutet und R' ein Halogenatom ist, mit der Maßgabe, daß außer bei spezieller Erwähnung die Reste Alkyl gerade oder verzweigt sind und 1 bis 4 Kohlenstoffatome enthalten,
cokristallisiert mit mindestens einer "Minderheits"-Komponente der Gruppe A der Streptogramine wie in Anspruch 1 definiert.

3. Gereinigte Form von Streptograminen nach Anspruch 1, dadurch gekennzeichnet, daß sie mindestens eine Komponente der Gruppe B der in der allgemeinen Formel (I) in Anspruch 1 definierten Streptogramine umfaßt, in der
- das Symbol R₁ einen Rest Ethyl darstellt, das Symbol R₂ ein Wasserstoffatom ist und
- das Symbol R₃ einen substituierten Rest Benzyl der allgemeinen Formel (III) bedeutet 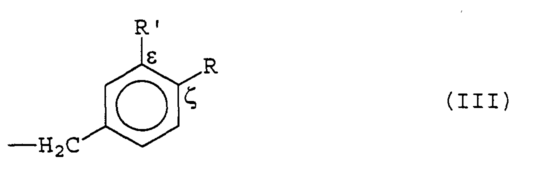
- in der
1) R NR₄R₅ darstellt, worin bei den Symbolen R₄ und R₅ das eine ein Wasserstoffatom oder ein Rest Methyl ist und das andere ein Rest Methyl ist und R' ein Chloratom oder Bromatom bedeutet oder einen Rest Allyl darstellt, wenn R₄ und R₅ Reste Methyl sind, oder
2) R ein Wasserstoffatom ist und R' einen Rest Alkylamino oder Dialkylamino, einen Rest Methoxy, Ethoxy, Allyloxy oder Trifluormethoxy oder einen Rest Alkylthio darstellt, oder
R einen Rest Alkylamino mit 2 bis 4 Kohlenstoffatomen in gerader oder verzweigter Kette, Dialkylamino, dessen Teile Alkyl gleich oder verschieden sind und 2 bis 4 Kohlenstoffatome in gerader oder verzweigter Kette enthalten, oder Methyl-ethyl-amino, einen Rest Pyrrolidino, einen Rest Allyl-alkyl-amino, dessen Teil Alkyl wie oben definiert ist, einen Rest Methoxy, Ethoxy, Allyloxy oder Trifluormethoxy, einen Rest Alkylthio, Alkyl mit 1 bis 6 Kohlenstoffatomen in gerader oder verzweigter Kette oder Trifluormethyl darstellt und R' ein Wasserstoffatom ist, oder
R einen Rest Alkyl mit 1 bis 6 Kohlenstoffatomen in gerader oder verzweigter Kette bedeutet und R' ein Halogenatom ist,
mit der Maßgabe, daß außer bei spezieller Erwähnung die Reste Alkyl gerade oder verzweigt sind und 1 bis 4 Kohlenstoffatome enthalten,
cokristallisiert mit mindestens einer "Minderheits"-Komponente der Gruppe A der Streptogramine wie in Anspruch 1 definiert.

4. Gereinigte Form von Streptograminen nach einem der Ansprüche 1 bis 3, dadurch gekennzeichnet, daß es sich um eine cokristallisierte Verbindung von einer oder von mehreren Komponenten der Gruppe B der wie in den Ansprüchen 1 bis 3 definierten Streptogramine und von einer oder von mehreren "Minderheits"-Komponenten der Gruppe A der wie in Anspruch 1 definierten Streptogramine mit einem molaren Verhältnis in der Größenordnung von 1/2 handelt.

5. Verfahren zur Herstellung einer gereinigten Form von Streptograminen nach einem der Ansprüche 1 bis 4, dadurch gekennzeichnet, daß man die Komponente(n) der Gruppe A der Streptogramine mit der (den) Komponente(n) der Gruppe B der Streptogramine, wie in den Ansprüchen 1 bis 3 definiert, cokristallisiert.

6. Gereinigte Komponente der Gruppe A der Streptogramine wie in Anspruch 1 definiert, wenn sie durch Vermittlung einer cokristallisierten Verbindung nach Anspruch 1 bis 4 erhalten wird.

7. Verwendung von einer oder von mehreren Minderheits-Komponenten der Gruppe A der Streptogramine wie in Anspruch 1 definiert zur Herstellung von Cokristallisaten mit einer oder mehreren Komponenten der Gruppe B der Streptogramine wie in Anspruch 1 bis 3 definiert.

8. Pharmazeutische Zusammensetzung, dadurch gekennzeichnet, daß sie eine gereinigte Form von Streptograminen nach einem der Ansprüche 1 bis 4 in reinem Zustand oder in Anwesenheit von jedem kompatiblen und pharmazeutisch akzeptablen Verdünnungsmittel oder Zusatzstoff enthält.

## Claims

1. Purified form of streptogramins, characterized in that it comprises at least one group B streptogramin component defined by the general formula: 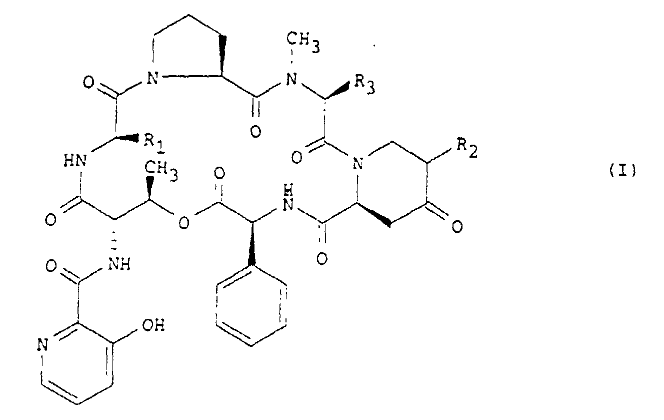 in which
- the symbol R₁ represents a methyl or ethyl radical, and
1) either
- the symbol R₂ represents a hydrogen atom and
- the symbol R₃ represents a substituted benzyl radical of general formula: 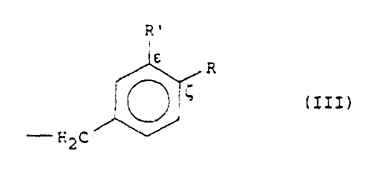 in which R represents NR₄R₅ for which the symbols R₄ and R₅ are one a hydrogen atom or a methyl radical and the other a methyl radical and R' is a chlorine or bromine atom, or represents an alkenyl radical containing 3 to 5 carbon atoms if R₄ and R₅ are methyl radicals,
2) or
- the symbol R₂ represents a hydrogen atom or a hydroxyl radical, and
- the symbol R₃ represents a radical of general formula (III)
in which R is a hydrogen atom and R' represents a halogen atom, an alkylamino or dialkylamino radical, the residue of an ether oxide, a radical alkylthio, alkyl containing 1 to 3 carbon atoms in a straight or branched chain or trihalomethyl, or alternatively
in which R is a halogen atom, an alkylamino radical containing 2 to 4 carbon atoms in a straight or branched chain, a dialkylamino radical in which the alkyl portions are identical or different and contain 2 to 4 carbon atoms in a straight or branched chain or a methylethylamino radical, a pyrrolidino radical, an alkenylalkylamino radical in which the alkenyl portion contains 3 or 4 carbon atoms and the alkyl portion is as defined above, a dialkenylamino radical in which the alkenyl portions are as defined above, an alkylcycloalkylmethylamino radical in which the alkyl portion is as defined above and the cycloalkyl portion contains 3 or 4 carbon atoms, the residue of an ether oxide, an alkylthio radical, a radical alkylthiomethyl, alkyl containing 1 to 6 carbon atoms in a straight or branched chain, aryl or trihalomethyl, and R' is a hydrogen atom, or alternatively
in which R is a halogen atom, an amino, alkylamino or dialkylamino radical, the residue of an ether oxide, a radical alkylthio, alkyl containing 1 to 6 carbon atoms in a straight or branched chain or trihalomethyl and R' represents a halogen atom, an alkylamino or dialkylamino radical, the residue of an ether oxide or an alkylthio or alkyl radical containing 1 to 3 carbon atoms in a straight or branched chain,
it being understood that, unless otherwise stated, the alkyl radicals are straight or branched and contain 1 to 4 carbon atoms,
cocrystallized with at least one "minor" group A streptogramin component of general formula: 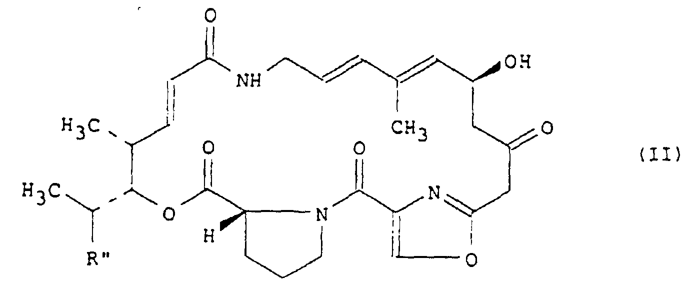 in which R" is a hydrogen atom or a methyl or ethyl radical.

2. Purified form of streptogramins according to Claim 1, characterized in that it comprises at least one group B streptogramin component defined by the general formula (I) in Claim 1, in which:
the symbol R₁ is as defined in Claim 1, and
1) either
the symbol R₂ represents a hydrogen atom and
the symbol R₃ represents a substituted benzyl radical of general formula: 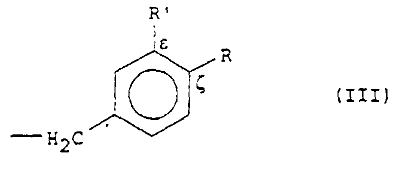 in which R represents NR₄R₅ for which the symbols R₄ and R₅ are one a hydrogen atom or a methyl radical and the other a methyl radical and R' is a chlorine or bromine atom, or represents an alkenyl radical containing 3 to 5 carbon atoms if R₄ and R₅ are methyl radicals,
2) or
the symbol R₂ represents a hydrogen atom or a hydroxyl radical, and
the symbol R₃ represents a radical of general formula (III)
in which R is a hydrogen atom and R' represents an alkylamino or dialkylamino radical, the residue of an ether oxide or an alkylthio radical, or alternatively in which R is an alkylamino radical containing 2 to 4 carbon atoms in a straight or branched chain, a dialkylamino radical in which the alkyl portions are identical or different and contain 2 to 4 carbon atoms in a straight or branched chain or a methylethylamino radical, a pyrrolidino radical, an alkenylalkylamino radical in which the alkenyl portion contains 3 or 4 carbon atoms and the alkyl portion is as defined above, the residue of an ether oxide, a radical alkylthio, alkyl containing 1 to 6 carbon atoms in a straight or branched chain or trihalomethyl, and R' is a hydrogen atom, or alternatively
in which R is an alkyl radical containing 1 to 6 carbon atoms in a straight or branched chain and R' represents a halogen atom,
it being understood that, unless otherwise stated, the alkyl radicals are straight or branched and contain 1 to 4 carbon atoms,
cocrystallized with at least one "minor" group A streptogramin component as defined in Claim 1.

3. Purified form of streptogramins according to Claim 1, characterized in that it comprises at least one group B streptogramin component defined by the general formula (I) in Claim 1, in which:
the symbol R₁ is an ethyl radical, the symbol R₂ is a hydrogen atom, and
the symbol R₃ represents a substituted benzyl radical of general formula: 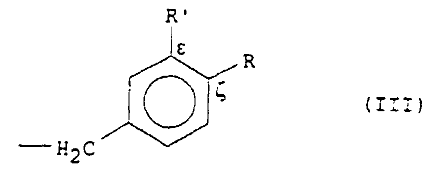
in which
1) R represents NR₄R₅ for which the symbols R₄ and R₅ are one a hydrogen atom or a methyl radical and the other a methyl radical and R' is a chlorine or bromine atom, or represents an allyl radical if R₄ and R₅ are methyl radicals, or alternatively
2) R is a hydrogen atom and R' represents an alkylamino or dialkylamino radical, a methoxy, ethoxy, allyloxy or trifluoromethoxy radical, or an alkylthio radical, or alternatively
R is an alkylamino radical containing 2 to 4 carbon atoms in a straight or branched chain, a dialkylamino radical in which the alkyl portions are identical or different and contain 2 to 4 carbon atoms in a straight or branched chain or a methylethylamino radical, a pyrrolidino radical, an allylalkylamino radical in which the alkyl portion is as defined above, a methoxy, ethoxy, allyloxy or trifluoromethoxy radical, a radical alkylthio, alkyl containing 1 to 6 carbon atoms in a straight or branched chain or trifluoromethyl, and R' is a hydrogen atom, or alternatively
R is an alkyl radical containing 1 to 6 carbon atoms in a straight or branched chain and R' represents a halogen atom,
it being understood that, unless otherwise stated, the alkyl radicals are straight or branched and contain 1 to 4 carbon atoms,
cocrystallized with at least one "minor" group A streptogramin component as defined in Claim 1.

4. Purified form of streptogramins according to one of Claims 1 to 3, characterized in that it is a compound cocrystallized from one or more group B streptogramin components defined in Claims 1 to 3 and from one or more minor group A streptogramin components as defined in Claim 1, in a molar ratio of the order of 1/2.

5. Process for the preparation of a purified form of streptogramins according to one of Claims 1 to 4, characterized in that the group A streptogramin component(s) is(are) cocrystallized with the group B streptogramin component(s) as defined in Claims 1 to 3.

6. Purified group A streptogramin component as defined in Claim 1, when it is obtained via a cocrystallized compound according to Claims 1 to 4.

7. Use of one or more minor group A streptogramin components as defined in Claim 1, for the preparation of cocrystallizates with one or more group B streptogramin components defined in Claims 1 to 3.

8. Pharmaceutical composition, characterized in that it contains a purified form of streptogramins according to one of Claims 1 to 4, in the pure state or in the presence of any compatible and pharmaceutically acceptable diluent or adjuvant.
